(19) Europäisches Patentamt
European Patent Office
Office européen des brevets

(11) EP 3 045 543 A2

(12) EUROPEAN PATENT APPLICATION

(43) Date of publication:
**20.07.2016 Bulletin 2016/29**

(51) Int Cl.:
***C12Q 1/68*** *(2006.01)*

(21) Application number: **15196262.8**

(22) Date of filing: **31.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.01.2012 US 201261592893 P**
**01.06.2012 US 201261654733 P**

(62) Document number(s) of the earlier application(s) in accordance with Art. 76 EPC:
**13704029.1 / 2 809 805**

(27) Previously filed application:
**31.01.2013 PCT/EP2013/051865**

(71) Applicant: **Novartis AG**
**4056 Basel (CH)**

(72) Inventor: **SPRAGGS, Colin F.**
**Stevenage**
**Hertfordshire SG1 2NY (GB)**

(74) Representative: **Gruber, Markus**
**Novartis Pharma AG**
**Patent Department**
**4002 Basel (CH)**

Remarks:
This application was filed on 25-11-2015 as a divisional application to the application mentioned under INID code 62.

# (54) METHOD OF TREATING CANCER

(57) Methods are provided for treating cancer in a patient in need thereof comprising; determining whether said patient has at least one polymorphism in VEGFA (rs3025039, VEGFA 936C>T), VEGFR (rs1870377, Q427H, VEGFR2 18487A>T, 1416A>T) or IGFIR (rs2037448, 229741A>G or rs7181022, 28322C>T); and if said patent has at least one polymorphism, administering to said patient a HER2 inhibitor.

EP 3 045 543 A2

**Description**

## Field of the Invention

The invention relates to methods for treating cancer with lapatinib, genetic markers useful in such treatment, and methods and reagents for detecting such genetic markers.

## Background of the Invention

Lapatinib is a dual HER2/EGFR tyrosine kinase inhibitor (TKI) approved in combination with capecitabine or letrozole for patients with HER2+ metastatic breast cancer (MBC). Consistent with HER2/EGFR and other TKI therapies, patient response is variable and suggestive of additional determinants of sensitivity and resistance. This exploratory pharmacogenetic study sought to identify host, germline genetic variants that associate with lapatinib treatment outcomes in HER2+ MBC patients.

Methods of treating patients with pharmacogenetic profiles that make them more likely to respond to treatment with pharmaceutical compounds are needed in clinical medicine.

## Summary of the Invention

In one embodiment, methods are provided for administering a HER2 inhibitor or to a patient in need thereof comprising:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, administering to said patient a HER2 inhibitor.

In one embodiment, methods are provided for prescribing a HER2 inhibitor to a patient in need thereof comprising:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, prescribing to said patient a HER2 inhibitor.

In one embodiment, methods are provided for treating cancer in a patient in need thereof comprises:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, administering to said patient an HER2 inhibitor.

## Brief Description of the Figures

Figure 1: VEGFA 936C>T and OS in Trial I.
Figure 2: VEGFR2 18487A>T (Q472H) and OS in Trial II (not Trial I).
Figure 3: IGFR1 rs2037448 & rs7181022 (tag SNPs, not functional) and OS in Trial II.
Figure 4A: NR1I3 (rs2307420) and PFS in meta-analysis
Figure 4B: VEGFA (936C>T, rs3025039) and OS in meta-analysis
Figure 4C: KDR/VEGFR2(18487T>A, Q472H, rs1870377) in meta-analysis
Figure 5: NR1I3 in PFS and OS
Figure 6: VEGFA (936C>T, rs3025039 and PFS and OS
Figure 7: KDR/VEGFR2(18487T>A, Q472H, rs1870377 and PFS and OS

## Detailed Description

Lapatinib is a HER2/EGFR tyrosine kinase inhibitor. Tyrosine kinase is associated with at least two oncogenes Epidermal Growth Factor Receptor (EGFR) and Human EGFR type 2 (Her2/neu). Overexpression of HER2/neu can be responsible for or correlated with certain types of high-risk breast cancers in women. Among other activities, lapatinib decreases tumor-causing breast cancer stem cells. One aspect of lapatinib's mechanism of action is that it inhibits receptor signal processes by binding to the ATP-binding pocket of the EGFR/HER2 protein kinase domain, preventing self-phosphorylation and subsequent activation of the signal mechanism.

Lapatinib is a small molecule and a member of the 4-anilinoquinazoline class of kinase inhibitors. In its currently marketed form, lapatinib is present as a monohydrate of the ditosylate salt, with chemical name N-(3 chloro-4-{[(3-fluorophenyl)methyl]oxy}phenyl)-6-[5-({[2 (methylsulfonyl)ethyl]amino}methyl)-2-furanyl]-4-quinazolinamine bis(4 methylbenzenesulfonate) monohydrate. It has the molecular formula $C_{29}H_{26}ClFN_4O_4S$ $(C_7H_8O_3S)_2$ $H_2O$ and a molecular weight of 943.5 daltons. Lapatinib Formula I has the following chemical structure:

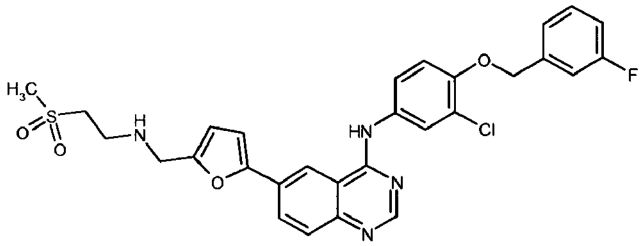

I

Lapatinib ditosylate monohydrate Formula I' has the following chemical structure:

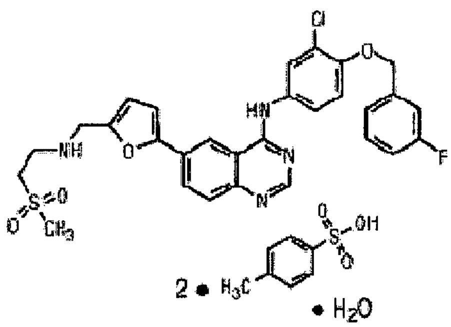

Lapatinib, pharmaceutically acceptable salt or compositions thereof, and compositions comprising lapatinib and uses are disclosed in, for example, US Patent Numbers 6,391,874, 6,828,320, US 6,727,256, 6,713,485, and 7,157,466.

Administering lapatinib, or a pharmaceutically acceptable salt or composition thereof, to a subject (or "treating" a subject with lapatinib) comprises methods and routes of administration as are known in the art. Recommended therapeutic regimes (dosing amounts and schedules, plasma concentrations) of lapatinib, and pharmaceutically acceptable salt or compositions thereof, are known in the art. As used herein, administration of lapatinib, or a pharmaceutically acceptable salt or composition thereof, is not limited to the treatment of breast cancer but includes its medical use for other conditions amenable to treatment with lapatinib, or pharmaceutically acceptable salt or compositions thereof.

As used herein, administration of a pharmaceutical kinase inhibitor to a subject comprises administration of an effective amount of the pharmaceutical agent to a subject in need thereof. The dose of a pharmaceutical agent can be determined according to methods known and accepted in the pharmaceutical arts, and can be determined by those skilled in the art.

As used herein, "genotyping" a subject (or DNA or other biological sample) for a polymorphic allele of a gene(s) means detecting which allelic or polymorphic form(s) of the gene(s) or gene expression products (e.g., hnRNA, mRNA or protein) are present or absent in a subject (or a sample). Related RNA or protein expressed from such gene may also be used to detect polymorphic variation. As is well known in the art, an individual may be heterozygous or homozygous for a particular allele. More than two allelic forms may exist, thus, there may be more than three possible genotypes. For purposes of the present invention, "genotyping" includes the determination of germline alleles using suitable techniques, as are known in the art. As used herein, an allele may be 'detected' when other possible allelic variants have

been ruled out; e.g., where a specified nucleic acid position is found to be neither adenine (A), thymine (T) or cytosine (C), it can be concluded that guanine (G) is present at that position (*i.e.,* G is 'detected' or 'diagnosed' in a subject). Sequence variations may be detected directly (by, e.g, sequencing) or indirectly (e.g., by restriction fragment length polymorphism analysis, or detection of the hybridization of a probe of known sequence, or reference strand conformation polymorphism), or by using other known methods.

As used herein, a "genetic subset" of a population consists of those members of the population having a particular genotype. In the case of a biallelic polymorphism, a population can potentially be divided into three subsets: homozygous for allele 1 (1,1), heterozygous (1,2), and homozygous for allele 2 (2,2). A 'population' of subjects may be defined using various criteria, *e.g.*, individuals being treated with lapatinib or individuals with cancer.

As used herein, a subject that is "predisposed to" or "at increased risk of" a particular phenotypic response based on genotyping will be more likely to display that phenotype than an individual with a different genotype at the target polymorphic locus (or loci). Where the phenotypic response is based on a multi-allelic polymorphism, or on the genotyping of more than one gene, the relative risk may differ among the multiple possible genotypes.

The term "wild type" as is understood in the art refers to a polypeptide or polynucleotide sequence that occurs in a native population without genetic modification. As is also understood in the art, a "variant" includes a polypeptide or polynucleotide sequence having at least one modification to an amino acid or nucleic acid compared to the corresponding amino acid or nucleic acid found in a wild type polypeptide or polynucleotide, respectively. Included in the term variant is Single Nucleotide Polymorphism (SNP) where a single base pair distinction exists in the sequence of a nucleic acid strand compared to the most prevalently found (wild type) nucleic acid strand. As used herein "genetic modification" or "genetically modified" refers to, but is not limited to, any suppression, substitution, deletion and/or insertion of one or more bases into DNA sequence(s). Also, as used herein "genetically modified" can refer to a gene encoding a polypeptide or a polypeptide having at least one deletion, substitution or suppression of a nucleic acid or amino acid, respectively.

Genetic variants and/or SNPs can be identified by known methods. For example, wild type or SNPs can be identified by DNA amplification and sequencing techniques, DNA and RNA detection techniques, including, but not limited to Northern and Southern blot, respectively, and/or various biochip and array technologies. WT and mutant polypeptides can be detected by a variety of techniques including, but not limited to immunodiagnostic techniques such as ELISA and western Blot.

Those skilled in the art will appreciate that polymorphisms which are similar to the [C/T] polymorphism shown in the sequence can also exist, namely [C/G] and [C/A]. When rs3025039 is used herein, it is meant to include the [C/T], [C/G], and [C/A] polymorphisms.

The rs3025039 reference single nucleotide polymorphisms was assayed by microarray using the Illumina Human 1M-Duo analysis BeadChip assay (http://www.illumina.com/products/humanlm_duo_dna_analysis_beadchip_kits.ilmn) In addition rs3025039 reference single nucleotide polymorphisms for which a sequence is known can be detected using various oligonucleotides as will be understood by those skilled in the art.

As used herein, "VEGF" means vascular endothelial growth factor.

As used herein, "VEGFR" means vascular endothelial growth factor receptor. The UniGene protein sequence references for VEGFR is: KDR (VEGFR2) NP_002244.

As used herein, "IGFR1" means insulin-like growth factor receptor 1. The UniGene protein sequence for IGFR1 is: IGF1R NP_000866.1.

As used herein, "VEGFA" means vascular endothelial growth factor A. (UniGene database states that the protein sequence ID is NP_001020537.2)

As used herein "EGFR" means epidermal growth factor receptor.

As used herin, "HER2" means HER2 (Human Epidermal Growth Factor Receptor 2) also known as Neu, ErbB-2, CD340 (cluster of differentiation 340) or p185 is a member of the epidermal growth factor receptor (EGFR/ErbB) family. The UniGene protein sequence for ERBB2 (HER2) is NP_004439.2.

An allele refers to one specific form of a genetic sequence (such as a gene) within a cell, a sample, an individual or within a population, the specific form differing from other forms of the same gene in the sequence of at least one, and frequently more than one, variant sites within the sequence of the gene. The sequences at these variant sites that differ between different alleles are termed "variants", "polymorphisms", or "mutations." In general, polymorphism is used to refer to variants that have a frequency of at least 1% in a population, while the term mutation is generally used for variants that occur at a frequency of less than 1% in a population. In diploid organisms such as humans, at each autosomal specific chromosomal location or "locus" an individual possesses two alleles, a first inherited from one parent and a second inherited from the other parent, for example one from the mother and one from the father. An individual is "heterozygous" at a locus if it has two different alleles at the locus. An individual is "homozygous" at a locus if it has two identical alleles at that locus.

A polymorphism may comprise one or more base changes, an insertion, a repeat, or a deletion. A polymorphic locus may be as small as one base pair. Polymorphic markers include restriction fragment length polymorphisms, variable

number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wild type form. The most frequent allele may also be referred to as the major allele and the less frequent allele as the minor allele. Diploid organisms may be homozygous or heterozygous for allelic forms. A diallelic polymorphism has two forms. A triallelic polymorphism has three forms. A polymorphism between two nucleic acids can occur naturally, or be caused by exposure to or contact with chemicals, enzymes, or other agents, or exposure to agents that cause damage to nucleic acids, for example, ultraviolet radiation, mutagens or carcinogens.

Single nucleotide polymorphisms (SNPs) are positions at which two alternative bases occur at appreciable frequency (>1%) in the human population, and are the most common type of human genetic variation. Approximately 90% of all polymorphisms in the human genome are SNPs. SNPs are single base positions in DNA at which different alleles, or alternative nucleotides, exist in a population. An individual may be homozygous or heterozygous for an allele at each SNP position. A SNP can, in some instances, be referred to as a "cSNP" to denote that the nucleotide sequence containing the SNP is an amino acid coding sequence. As used herein, references to SNPs and SNP genotypes include individual SNPs and/or haplotypes, which are groups of SNPs that are generally inherited together. Haplotypes can have stronger correlations with diseases or other phenotypic effects compared with individual SNPs, and therefore may provide increased diagnostic accuracy in some cases (Stephens et al. Science 293, 489-493, 20 Jul. 2001).

Causative SNPs are those SNPs that produce alterations in gene expression or in the expression, structure, and/or function of a gene product, and therefore are most predictive of a possible clinical phenotype. One such class includes SNPs falling within regions of genes encoding a polypeptide product, i.e. cSNPs. These SNPs may result in an alteration of the amino acid sequence of the polypeptide product (i.e., non-synonymous codon changes) and give rise to the expression of a defective or other variant protein. Furthermore, in the case of nonsense mutations, a SNP may lead to premature termination of a polypeptide product. Causative SNPs do not necessarily have to occur in coding regions; causative SNPs can occur in, for example, any genetic region that can ultimately affect the expression, structure, and/or activity of the protein encoded by a nucleic acid. Such genetic regions include, for example, those involved in transcription, such as SNPs in transcription factor binding domains, SNPs in promoter regions, in areas involved in transcript processing, such as SNPs at intron-exon boundaries that may cause defective splicing, or SNPs in mRNA processing signal sequences such as polyadenylation signal regions. Some SNPs that are not causative SNPs nevertheless are in close association with, and therefore segregate with, a disease-causing sequence. In this situation, the presence of a SNP correlates with the presence of, or predisposition to, or an increased risk in developing the disease. These SNPs, although not causative, are nonetheless also useful for diagnostics, disease predisposition screening, and other uses.

An association study of a SNP and a specific disorder or a predisposition to a safety event or therapeutic outcome involves determining the presence or frequency of the SNP allele in biological samples from individuals with the disorder or predisposition to a safety event of interest and comparing the information to that of controls (i.e., individuals who do not have the disorder or experience the same safety event or therapeutic outcome).

A SNP may be screened in diseased tissue samples or any biological sample obtained from an individual, and compared to control samples, and selected for its increased (or decreased) occurrence in a specific pathological condition. Once a statistically significant association is established between one or more SNP(s) and a pathological condition (or other phenotype) of interest, then the region around the SNP can optionally be thoroughly screened to identify the causative genetic locus/sequence(s) (e.g., causative SNP/mutation, gene, regulatory region, etc.) that influences the pathological condition or phenotype.

Clinical trials have shown that patient response to treatment with pharmaceuticals is often heterogeneous. There is a continuing need to improve pharmaceutical agent design and therapy. In that regard, SNPs can be used to identify patients most suited to therapy with particular pharmaceutical agents (this is often termed "pharmacogenomics"). Similarly, SNPs can be used to exclude patients from certain treatment due to the patient's increased likelihood of developing toxic side effects or their likelihood of not responding to the treatment. Pharmacogenomics can also be used in pharmaceutical research to assist the drug development and selection process. (Linder et al. (1997), Clinical Chemistry, 43, 254; Marshall (1997), Nature Biotechnology, 15, 1249; International Patent Application WO 97/40462, Spectra Biomedical; and Schafer et al. (1998), Nature Biotechnology, 16, 3).

Several techniques for the detection of mutations have evolved based on the principal of hybridization analysis. For example, in the primer extension assay, the DNA region spanning the nucleotide of interest is amplified by PCR, or any other suitable amplification technique. After amplification, a primer is hybridized to a target nucleic acid sequence, wherein the last nucleotide of the 3' end of the primer anneals immediately 5' to the nucleotide position on the target sequence that is to be analyzed. The annealed primer is extended by a single, labelled nucleotide triphosphate. The incorporated nucleotide is then detected.

The sequence of any nucleic acid including a gene or PCR product or a fragment or portion thereof may be

sequenced by any method known in the art (e.g., chemical sequencing or enzymatic sequencing). "Chemical sequencing" of DNA may denote methods such as that of Maxam and Gilbert (1977) (Proc. Natl. Acad. Sci. USA 74:560), in which DNA is randomly cleaved using individual base-specific reactions. "Enzymatic sequencing" of DNA may denote methods such as that of Sanger (Sanger, et al., (1977) Proc. Natl. Acad. Sci. USA 74:5463).

Conventional molecular biology, microbiology, and recombinant DNA techniques including sequencing techniques are well known among those skilled in the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, N.Y. (herein "Sambrook, et al., 1989"); DNA Cloning: A Practical Approach, Volumes I and II (D. N. Glover ed. 1985); Oligonucleotide Synthesis (M. J. Gait ed. 1984); Nucleic Acid Hybridization (B. D. Hames & S. J. Higgins eds. (1985)); Transcription And Translation (B. D. Hames & S. J. Higgins, eds. (1984)); Animal Cell Culture (R. I. Freshney, ed. (1986)); Immobilized Cells And Enzymes (IRL Press, (1986)); B. Perbal, A Practical Guide To Molecular Cloning (1984); F. M. Ausubel, et al. (eds.), Current Protocols in Molecular Biology, John Wiley & Sons, Inc. (1994

The Peptide Nucleic Acid (PNA) affinity assay is a derivative of traditional hybridization assays (Nielsen et al., Science 254:1497-1500 (1991); Egholm et al., J. Am. Chem. Soc. 114:1895-1897 (1992); James et al., Protein Science 3:1347-1350 (1994)). PNAs are structural DNA mimics that follow Watson-Crick base pairing rules, and are used in standard DNA hybridization assays. PNAs display greater specificity in hybridization assays because a PNA/DNA mismatch is more destabilizing than a DNA/DNA mismatch and complementary PNA/DNA strands form stronger bonds than complementary DNA/DNA strands.

DNA microarrays have been developed to detect genetic variations and polymorphisms (Taton et al., Science 289:1757-60, 2000; Lockhart et al., Nature 405:827-836 (2000); Gerhold et al., Trends in Biochemical Sciences 24:168-73 (1999); Wallace, R. W., Molecular Medicine Today 3:384-89 (1997); Blanchard and Hood, Nature Biotechnology 149:1649 (1996)). DNA microarrays are fabricated by high-speed robotics, on glass or nylon substrates, and contain DNA fragments with known identities ("the probe"). The microarrays are used for matching known and unknown DNA fragments ("the target") based on traditional base-pairing rules.

The Protein Truncation Test (PTT) is also commonly used to detect genetic polymorphisms (Roest et al., Human Molecular- Genetics 2:1719-1721, (1993); Van Der Luit et al., Genomics 20:1-4 (1994); Hogervorst et al., Nature Genetics 10: 208-212 (1995)). Typically, in the PTT, the gene of interest is PCR amplified, subjected to *in vitro* transcription/translation, purified, and analyzed by polyacrylamide gel electrophoresis.

"Genetic testing" (also called genetic screening) as used herein refers to the testing of a biological sample from a subject to determine the subject's genotype; and may be utilized to determine if the subject's genotype comprises alleles that either cause, or increase susceptibility to, a particular phenotype (or that are in linkage disequilibrium with allele(s) causing or increasing susceptibility to that phenotype).

"Linkage disequilibrium" refers to the tendency of specific alleles at different genomic locations to occur together more frequently than would be expected by chance. Alleles at given loci are in complete equilibrium if the frequency of any particular set of alleles (or haplotype) is the product of their individual population frequencies A commonly used measure of linkage disequilibrium is r:

$$r = \frac{\hat{\Delta}_{AB}}{\sqrt{(\tilde{\pi}_A + \hat{D}_A)(\tilde{\pi}_B + \hat{D}_B)}}$$

where

$$\tilde{\pi}_A = \tilde{p}_A(1\text{-}\tilde{p}_A),\ \tilde{\pi}_B = \tilde{p}_B(1\text{-}\tilde{p}_B),\ \hat{D}_A = \tilde{P}_{AA} - \tilde{p}_A^2,\ \hat{D}_B = \tilde{P}_{BB} - \tilde{p}_B^2$$

$$\hat{\Delta}_{AB} = \frac{1}{n} n_{AB} \text{ -}2\tilde{p}_A\tilde{p}_B$$

$nr^2$ has an approximate chi square distribution with 1 degree freedom for biallelic markers. Loci exhibiting an *r* such that $nr^2$ is greater than 3.84, corresponding to a significant chi-squared statistic at the 0.05 level, are considered to be in linkage disequilibrium (BS Weir 1996 Genetic Data Analysis II Sinauer Associates, Sunderland, MD).

Alternatively, a normalized measure of linkage disequilibrium can be defined as:

$$D'_{AB} = \begin{cases} \dfrac{D_{AB}}{\min(p_A p_B, p_a p_b)}, & D_{AB} < 0 \\ \dfrac{D_{AB}}{\min(p_A p_b, p_a p_B)}, & D_{AB} > 0 \end{cases}$$

The value of the D' has a range of -1.0 to 1.0. When statistically significant absolute D' value for two markers is not less than 0.3 they are considered to be in linkage disequilibrium.

As used herein the word "haplotype" refers to a set of closely linked alleles present on one chromosome which tend to be inherited together. A VEGFA genotype can be identified by detecting the presence of a VEGFA allele, or detecting a genetic marker known to be in linkage disequilibrium with a VEGFA allele. A genotype refers to variation at a defined position in a single gene, eg, 1,1 1,2 2,2.

As used herein, determination of a 'multilocus' genotype (also known as a haplotype) refers to the detection within an individual of the alleles present at more than one locus.

As used herein, the process of detecting an allele or polymorphism includes but is not limited to genetic methods. The allele or polymorphism detected may be functionally involved in affecting an individual's phenotype, or it may be an allele or polymorphism that is in linkage disequilibrium with a functional polymorphism/allele. Polymorphisms/alleles are evidenced in the genomic DNA of a subject, but may also be detectable from RNA, cDNA or protein sequences transcribed or translated from this region, as will be apparent to one skilled in the art.

In another embodiment of the present invention, lapatinib, or a pharmaceutically acceptable salt or composition thereof, is administered to said human as monotherapy. In another embodiment, lapatinib, or a pharmaceutically acceptable salt or composition thereof, is administered with at least one other anti-neoplastic agent. The one other anti-neoplastic agent may be selected from, but not limited to, the group of: trastuzumab, pertuzumab, capecitabine, paclitaxel, carboplatin, pazopanib and letrozole.

Methods of the invention may be used with human subjects diagnosed with or suffering from any cancer, including but not limited to cancer that is susceptible to inhibition of EGFR, HER2/erbB-2, VEGF, VEGFR and intracellular transducing proteins including, but not limited to PI3K, Akt, and mTOR as well as both primary and metastatic forms of head and neck, breast, lung, colon, ovary, and prostate cancers. The methods may also be used for any human subject being treated with lapatinib.

Polymorphic alleles may be detected by determining the DNA polynucleotide sequence, or by detecting the corresponding sequence in RNA transcripts from the polymorphic gene, or where the nucleic acid polymorphism results in a change in an encoded protein by detecting such amino acid sequence changes in encoded proteins; using any suitable technique as is known in the art. Polynucleotides utilized for typing are typically genomic DNA, or a polynucleotide fragment derived from a genomic polynucleotide sequence, such as in a library made using genomic material from the individual (e.g. a cDNA library). The polymorphism may be detected in a method that comprises contacting a polynucleotide or protein sample from an individual with a specific binding agent for the polymorphism and determining whether the agent binds to the polynucleotide or protein, where the binding indicates that the polymorphism is present. The binding agent may also bind to flanking nucleotides and amino acids on one or both sides of the polymorphism, for example at least 2, 5, 10, 15 or more flanking nucleotide or amino acids in total or on each side. In the case where the presence of the polymorphism is being determined in a polynucleotide it may be detected in the double stranded form, but is typically detected in the single stranded form.

The binding agent may be a polynucleotide (single or double stranded) typically with a length of at least 10 nucleotides, for example at least 15, 20, 30, or more nucleotides. A polynucleotide agent which is used in the method will generally bind to the polymorphism of interest, and the flanking sequence, in a sequence specific manner (e.g. hybridize in accordance with Watson-Crick base pairing) and thus typically has a sequence which is fully or partially complementary to the sequence of the polymorphism and flanking region. The binding agent may be a molecule that is structurally similar to polynucleotides that comprises units (such as purine or pyrimidine analogs, peptide nucleic acids, or RNA derivatives such as locked nucleic acids (LNA)) able to participate in Watson-Crick base pairing. The agent may be a protein, typically with a length of at least 10 amino acids, such as at least 20, 30, 50, or 100 or more amino acids. The agent may be an antibody (including a fragment of such an antibody that is capable of binding the polymorphism).

In one embodiment of the present methods a binding agent is used as a probe. The probe may be labelled or may be capable of being labelled indirectly. The detection of the label may be used to detect the presence of the probe on (bound to) the polynucleotide or protein of the individual. The binding of the probe to the polynucleotide or protein may be used to immobilize either the probe or the polynucleotide or protein (and, thus, to separate it from one composition or solution).

In another embodiment of the invention the polynucleotide or protein of the individual is immobilized on a solid support and then contacted with the probe. The presence of the probe immobilized to the solid support (via its binding

to the polymorphism) is then detected, either directly by detecting a label on the probe or indirectly by contacting the probe with a moiety that binds the probe. In the case of detecting a polynucleotide polymorphism the solid support is generally made of nitrocellulose or nylon. In the case of a protein polymorphism the method may be based on an ELISA system.

The present methods may be based on an oligonucleotide ligation assay in which two oligonucleotide probes are used. These probes bind to adjacent areas on the polynucleotide which contains the polymorphism, allowing (after binding) the two probes to be ligated together by an appropriate ligase enzyme. However the two probes will only bind (in a manner which allows ligation) to a polynucleotide that contains the polymorphism, and therefore the detection of the ligated product may be used to determine the presence of the polymorphism.

In one embodiment the probe is used in a heteroduplex analysis based system to detect polymorphisms. In such a system when the probe is bound to a polynucleotide sequence containing the polymorphism, it forms a heteroduplex at the site where the polymorphism occurs (i.e. it does not form a double strand structure). Such a heteroduplex structure can be detected by the use of an enzyme that is single or double strand specific. Typically the probe is an RNA probe and the enzyme used is RNAse H that cleaves the heteroduplex region, thus, allowing the polymorphism to be detected by means of the detection of the cleavage products.

The method may be based on fluorescent chemical cleavage mismatch analysis which is described for example in PCR Methods and Applications 3:268-71 (1994) and Proc. Natl. Acad. Sci. 85:4397-4401 (1998).

In one embodiment the polynucleotide agent is able to act as a primer for a PCR reaction only if it binds a polynucleotide containing the polymorphism (i.e. a sequence- or allele-specific PCR system). Thus, a PCR product will only be produced if the polymorphism is present in the polynucleotide of the individual, and the presence of the polymorphism is determined by the detection of the PCR product. Preferably the region of the primer which is complementary to the polymorphism is at or near the 3' end the primer. In one embodiment of this system the polynucleotide the agent will bind to the wild-type sequence but will not act as a primer for a PCR reaction.

The method may be a Restriction Fragment Length Polymorphism (RFLP) based system. This can be used if the presence of the polymorphism in the polynucleotide creates or destroys a restriction site that is recognized by a restriction enzyme. Thus, treatment of a polynucleotide that has such a polymorphism will lead to different products being produced compared to the corresponding wild-type sequence. Thus, the detection of the presence of particular restriction digest products can be used to determine the presence of the polymorphism.

The presence of the polymorphism may be determined based on the change that the presence of the polymorphism makes to the mobility of the polynucleotide or protein during gel electrophoresis. In the case of a polynucleotide single-stranded conformation polymorphism (SSCP) analysis may be used. This measures the mobility of the single stranded polynucleotide on a denaturing gel compared to the corresponding wild-type polynucleotide, the detection of a difference in mobility indicating the presence of the polymorphism. Denaturing gradient gel electrophoresis (DGGE) is a similar system where the polynucleotide is electrophoresed through a gel with a denaturing gradient, a difference in mobility compared to the corresponding wild-type polynucleotide indicating the presence of the polymorphism.

The presence of the polymorphism may be determined using a fluorescent dye and quenching agent-based PCR assay such as the TAQMAN™ PCR detection system. In another method of detecting the polymorphism a polynucleotide comprising the polymorphic region is sequenced across the region which contains the polymorphism to determine the presence of the polymorphism.

Various other detection techniques suitable for use in the present methods will be apparent to those conversant with methods of detecting, identifying, and/or distinguishing polymorphisms. Such detection techniques include but are not limited to direct sequencing, use of "molecular beacons" (oligonucleotide probes that fluoresce upon hybridization, useful in real-time fluorescence PCR; see e.g., Marras et al., Genet Anal 14:151 (1999)); electrochemical detection (reduction or oxidation of DNA bases or sugars; see US Patent No. 5,871,918 to Thorp et al.); rolling circle amplification (see, e.g., Gusev et al., Am J Pathol 159:63 (2001)); Third Wave Technologies (Madison WI) INVADER® non-PCR based detection method (see, e.g., Lieder, Advance for Laboratory Managers, 70 (2000))

Accordingly, any suitable detection technique as is known in the art may be utilized in the present methods.

As used herein, "determining" a subject's genotype does not require that a genotyping technique be carried out where a subject has previously been genotyped and the results of the previous genetic test are available; determining a subject's genotype accordingly includes referring to previously completed genetic analyses.

The present invention also provides for a predictive (patient care) test or test kit. Such a test will aid in the therapeutic use of pharmaceutical compounds, including tyrosine kinase inhibitors, such as lapatinib, based on predetermined associations between genotype and phenotypic response to the therapeutic compound. Such a test may take different formats, including:

(a) a test which analyzes DNA or RNA for the presence of pre-determined alleles and/or polymorphisms. An appropriate test kit may include one or more of the following reagents or instruments: an enzyme able to act on a polynucleotide (typically a polymerase or restriction enzyme), suitable buffers for enzyme reagents, PCR primers which

bind to regions flanking the polymorphism, a positive or negative control (or both), and a gel electrophoresis apparatus. The product may utilise one of the chip technologies as described by the state of the art. The test kit would include printed or machine readable instructions setting forth the correlation between the presence of a specific genotype and the likelihood that a subject treated with a specific pharmaceutical compound will experience a hypersensitivity reaction;

(b) a test which analyses materials derived from the subject's body, such as proteins or metabolites, that indicate the presence of a pre-determined polymorphism or allele. An appropriate test kit may comprise a molecule, aptamer, peptide or antibody (including an antibody fragment) that specifically binds to a predetermined polymorphic region (or a specific region flanking the polymorphism). The kit may additionally comprise one or more additional reagents or instruments (as are known in the art). The test kit would also include printed or machine-readable instructions setting forth the correlation between the presence of a specific polymorphism or genotype and the likelihood that a subject treated with a specific synthetic nucleoside analog will experience a defined phenotype, reaction or clinical outcome.

Suitable biological specimens for testing are those which comprise cells and DNA and include, but are not limited to blood or blood components, dried blood spots, urine, buccal swabs and saliva. Suitable samples for genetic and peptide/protein testing are well known in the art.

Typically, any anti-neoplastic agent that has activity versus a susceptible tumor being treated may be co-administered in the treatment of cancer in the present invention. Examples of such agents can be found in Cancer Principles and Practice f Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; and cell cycle signaling inhibitors.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti - cancer agents that operate at the $G_2$/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree *Taxus brevifolia* and is commercially available as an injectable solution TAXOL®. It is a member of the taxane family of terpenes. It was first isolated in 1971 by Wani et al. J. Am. Chem, Soc., 93:2325. 1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77:1561-1565 (1980); Schiff et al., Nature, 277:665-667 (1979); Kumar, J. Biol, Chem, 256: 10435-10441 (1981). For a review of synthesis and anticancer activity of some paclitaxel derivatives see: D. G. I. Kingston et al., Studies in Organic Chemistry vol. 26, entitled "New trends in Natural Products Chemistry 1986", Attaur-Rahman, P.W. Le Quesne, Eds. (Elsevier, Amsterdam, 1986) pp 219-235.

Paclitaxel has been approved for clinical use in the treatment of refractory ovarian cancer in the United States (Markman et al., Yale Journal of Biology and Medicine, 64:583, 1991; McGuire et al., Ann. Intem, Med., 111:273,1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994, lung cancer and malaria. Treatment of patients with paclitaxel results in bone marrow suppression (multiple cell lineages, Ignoff, R.J. et. al, Cancer Chemotherapy Pocket Guide, 1998) related to the duration of dosing above a threshold concentration (50nM) (Kearns, C.M. et. al., Seminars in Oncology, 3(6) p.16-23, 1995).

Docetaxel, (2R,3S)-N-carboxy-3-phenylisoserine,N-*tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel *q.v.,* prepared using a natural precursor, 10-deacetyl-baccatin III, extracted from the needle of

the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as PLATINOL® as an injectable solution. Cisplatin is primarily indicated in the treatment of metastatic testicular and ovarian cancer and advanced bladder cancer. The primary dose limiting side effects of cisplatin are nephrotoxicity, which may be controlled by hydration and diuresis, and ototoxicity.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced ovarian carcinoma. Bone marrow suppression is the dose limiting toxicity of carboplatin.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfllydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias. Alopecia, nausea, vomiting and leukopenia are the most common dose limiting side effects of cyclophosphamide.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease. Bone marrow suppression is the most common dose limiting side effect of chlorambucil.

Busulfan, 1,4-butanediol dimethanesulfonate, is commercially available as MYLERAN® TABLETS. Busulfan is indicated for the palliative treatment of chronic myelogenous leukemia. Bone marrow suppression is the most common dose limiting side effects of busulfan.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease. Nausea, vomiting, and anorexia are the most common dose limiting side effects of dacarbazine.

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also know as Actinomycin D, is commercially available in injectable form as COSMEGEN®. Dactinomycin is indicated for the treatment of Wilm's tumor and rhabdomyosarcoma. Nausea, vomiting, and anorexia are the most common dose limiting side effects of dactinomycin.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma. Myelosuppression is the most common dose limiting side effect of daunorubicin.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas. Myelosuppression is the most common dose limiting side effect of doxorubicin.

Bleomycin, a mixture of cytotoxic glycopeptide antibiotics isolated from a strain of *Streptomyces verticillus,* is commercially available as BLENOXANE®. Bleomycin is indicated as a palliative treatment, as a single agent or in combination with other agents, of squamous cell carcinoma, lymphomas, and testicular carcinomas. Pulmonary and cutaneous toxicities are the most common dose limiting side effects of bleomycin.

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and $G_2$ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as a single agent or in combination with other chemotherapy agents in the treatment of testicular and non-small cell lung cancers. Myelosuppression is the most common side effect of etoposide. The incidence of leucopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON® and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leucopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2 (1H)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leucopenia, thrombocytopenia, and mucositis.

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppression and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID®. Thioguanine

exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leucopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Gemcitabine, 2'-deoxy-2',2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer. Myelosuppression, including leucopenia, thrombocytopenia, and anemia, is the most common dose limiting side effect of gemcitabine administration.

Methotrexate, N-[4[[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder. Myelosuppression (leucopenia, thrombocytopenia, and anemia) and mucositis are expected side effect of methotrexate administration.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethytenedioxy-20-camptothecin described below.

Irinotecan HCl, (4S)-4,11-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR®.

Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I: DNA : irintecan or SN-38 ternary complex with replication enzymes. Irinotecan is indicated for treatment of metastatic cancer of the colon or rectum. The dose limiting side effects of irinotecan HCl are myelosuppression, including neutropenia, and GI effects, including diarrhea.

Topotecan HCl, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer. The dose limiting side effect of topotecan HCl is myelosuppression, primarily neutropenia.

Rituximab is a chimeric monoclonal antibody which is sold as RITUXAN® and MABTHERA®. Rituximab binds to CD20 on B cells and causes cell apoptosis. Rituximab is administered intravenously and is approved for treatment of rheumatoid arthritis and B-cell non-Hodgkin's lymphoma.

Ofatumumab is a fully human monoclonal antibody which is sold as ARZERRA®. Ofatumumab binds to CD20 on B cells and is used to treat chronic lymphocytic leukemia CLL; a type of cancer of the white blood cells) in adults who are refractory to treatment with fludarabine (Fludara) and alemtuzumab Campath).

Trastuzumab (HEREPTIN®) is a humanized monoclonal antibody that binds to the HER2 receptor. It original indication is HER2 positive breast cancer.

Cetuximab (ERBITUX®) is a chimeric mouse human antibody that inhibits epidermal growth factor receptor (EGFR).

Pertuzumab (also called 2C4, trade name Omnitarg) is a monoclonal antibody. The first of its class in a line of agents called "HER dimerization inhibitors". By binding to HER2, it inhibits the dimerization of HER2 with other HER receptors, which is hypothesized to result in slowed tumor growth. Pertuzumab is described in WO01/00245 published January 4, 2001.

mTOR inhibitors include but are not limited to rapamycin (FK506) and rapalogs, RAD001 or everolimus (Afinitor), CCI-779 or temsirolimus, AP23573, AZD8055, WYE-354, WYE-600, WYE-687 and Pp121.

Bexarotene is sold as Targretin® and is a member of a subclass of retinoids that selectively activate retinoid X receptors (RXRs). These retinoid receptors have biologic activity distinct from that of retinoic acid receptors (RARs). The chemical name is 4-[1-(5,6,7,8-tetrahydro-3,5,5,8,8-pentamethyl-2-naphthalenyl) ethenyl] benzoic acid. Bexarotene is used to treat cutaneous T-cell lymphoma CTCL, a type of skin cancer) in people whose disease could not be treated successfully with at least one other medication.

Sorafenib marketed as Nexavar® is in a class of medications called multikinase inhibitors. Its chemical name is 4-[4-[[4-chloro-3-(trifluoromethyl)phenyl]carbamoylamino] phenoxy]-*N*-methyl-pyridine-2-carboxamide. Sorafenib is

used to treat advanced renal cell carcinoma (a type of cancer that begins in the kidneys). Sorafenib is also used to treat unresectable hepatocellular carcinoma (a type of liver cancer that cannot be treated with surgery).

Examples of erbB inhibitors include lapatinib, erlotinib, and gefitinib. Lapatinib, *N*-(3-chloro-4-{[(3-fluorophenyl)methyl]oxy}phenyl)-6-[5-({[2-(methylsulfonyl)ethyl]amino}methyl)-2-furanyl]-4-quinazolinamine (represented by Formula I, as illustrated), is a potent, oral, small-molecule, dual inhibitor of erbB-1 and erbB-2 (EGFR and HER2) tyrosine kinases that is approved in combination with capecitabine for the treatment of HER2-positive metastatic breast cancer.

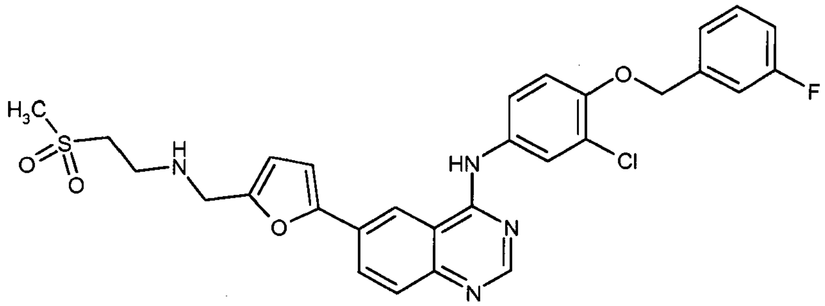

I

The free base, HCl salts, and ditosylate salts of the compound of formula (I) may be prepared according to the procedures disclosed in WO 99/35146, published July 15, 1999; and WO 02/02552 published January 10, 2002.

Erlotinib, *N*-(3-ethynylphenyl)-6,7-bis{[2-(methyloxy)ethyl]oxy}-4-quinazolinamine Commercially available under the tradename Tarceva) is represented by formula II, as illustrated:

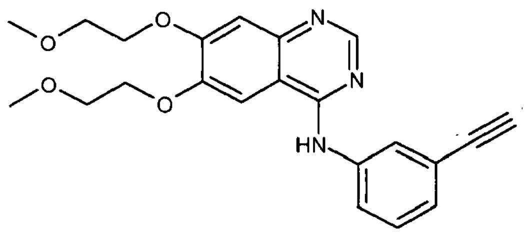

II

The free base and HCl salt of erlotinib may be prepared, for example, according to U.S. 5,747,498, Example 20.

Gefitinib, 4-quinazolinamine,N-(3-chloro-4-fluorophenyl)-7-methoxy-6-[3-4-morpholin)propoxy] is represented by formula III, as illustrated:

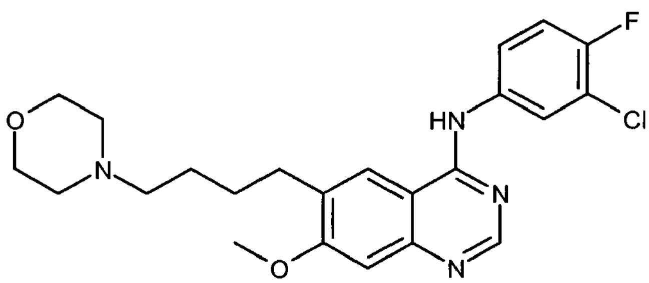

III

Gefitinib, which is commercially available under the trade name IRESSA® (Astra-Zenenca) is an erbB-1 inhibitor that is indicated as monotherapy for the treatment of patients with locally advanced or metastatic non-small-cell lung cancer after failure of both platinum-based and docetaxel chemotherapies. The free base, HCl salts, and diHCl salts of

gefitinib may be prepared according to the procedures of International Patent Application No. PCT/GB96/00961, filed April 23, 1996, and published as WO 96/33980 on October 31, 1996.

Also of interest, is the camptothecin derivative of formula A following, currently under development, including the racemic mixture (R,S) form as well as the R and S enantiomers:

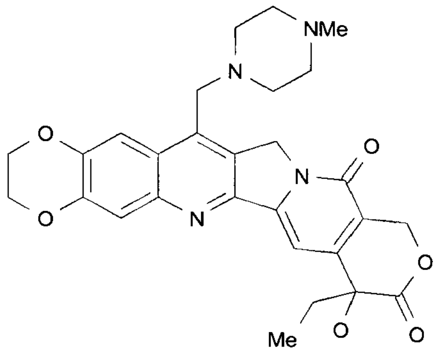

A

known by the chemical name "7-(4-methylpiperazino-methylene)-10,11-ethylenedioxy-20(R,S)-camptothecin (racemic mixture) or "7-(4-methylpiperazino-methylene)-10,11-ethylenedioxy-20(R)-camptothecin (R enantiomer) or "7-(4-methylpiperazino-methylene)-10,11-ethylenedioxy-20(S)-camptothecin (S enantiomer). Such compound as well as related compounds are described, including methods of making, in U.S. Patent Nos. 6,063,923; 5,342,947; 5,559,235; 5,491,237 and pending U.S. patent Application No. 08/977,217 filed November 24, 1997.

Hormones and hormonal analogues are useful compounds for treating cancers in which there is a relationship between the hormone(s) and growth and/or lack of growth of the cancer. Examples of hormones and hormonal analogues useful in cancer treatment include, but are not limited to, adrenocorticosteroids such as prednisone and prednisolone which are useful in the treatment of malignant lymphoma and acute leukemia in children; aminoglutethimide and other aromatase inhibitors such as anastrozole, letrozole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and anti-androgens such as flutamide, nilutamide, bicalutamide, cyproterone acetate and 5α-reductases such as finasteride and dutasteride, useful in the treatment of prostatic carcinoma and benign prostatic hypertrophy; antiestrogens such as tamoxifen, toremifene, raloxifene, droloxifene, iodoxyfene, as well as selective estrogen receptor modulators (SERMS) such those described in U.S. Patent Nos. 5,681,835, 5,877,219, and 6,207,716, useful in the treatment of hormone dependent breast carcinoma and other susceptible cancers; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

Letrozole (trade name Femara) is an oral non-steroidal aromatase inhibitor for the treatment of hormonally-responsive breast cancer after surgery. Estrogens are produced by the conversion of androgens through the activity of the aromatase enzyme. Estrogens then bind to an estrogen receptor, which causes cells to divide. Letrozole prevents the aromatase from producing estrogens by competitive, reversible binding to the heme of its cytochrome P450 unit. The action is specific, and letrozole does not reduce production of mineralo- or corticosteroids.

Signal transduction pathway inhibitors are those inhibitors, which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3domain blockers, serine/threonine kinases, phosphotidyl inositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are generally termed growth factor receptors. Inappropriate or uncontrolled activation of many of these kinases, i.e. aberrant kinase growth factor receptor activity, for example by overexpression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr), platelet derived growth factor receptor (PDGFr), erbB2, erbB4, vascular endothelial growth factor receptor (VEGFr), tyrosine kinase with immunoglobulin-like and epidermal growth factor homology domains (TIE-2), insulin growth factor -I (IGFI) receptor, macrophage colony stimulating factor Cfms), BTK, ckit, cmet, fibroblast growth factor (FGF) receptors, Trk receptors (TrkA, TrkB, and TrkC), ephrin (eph) receptors, and the RET protooncogene. Several inhibitors of growth receptors are under development and include ligand antagonists, antibodies, tyrosine kinase inhibitors and anti-sense oligonucleotides. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth factor receptors as targets", New Molecular Targets for Cancer Chemotherapy, ed. Workman, Paul and Kerr, David, CRC press 1994, London.

Tyrosine kinases, which are not growth factor receptor kinases are termed non-receptor tyrosine kinases. Non-receptor tyrosine kinases useful in the present invention, which are targets or potential targets of anti-cancer drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997 Annual review of Immunology. 15: 371-404.

SH2/SH3 domain blockers are agents that disrupt SH2 or SH3 domain binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (rafk), Mitogen or Extracellular Regulated Kinase (MEKs), and Extracellular Regulated Kinases (ERKs); and Protein kinase C family member blockers including blockers of PKCs (alpha, beta, gamma, epsilon, mu, lambda, iota, zeta). IkB kinase family (IKKa, IKKb), PKB family kinases, AKT kinase family members, and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60. 1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; U.S. Patent No. 6,268,391; and Martinez-lacaci, L., et al, Int. J. Cancer (2000), 88(1), 44-52.

Inhibitors of Phosphotidyl inositol-3 Kinase family members including blockers of PI3-kinase, ATM, DNA-PK, and Ku are also useful in the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997, International Journal of Biochemistry and Cell Biology. 29 (7:935-8; and Zhong, H. et al, Cancer res, (2000) 60(6), 1541-1545.

Also useful in the present invention are Myo-inositol signaling inhibitors such as phospholipase C blockers and Myoinositol analogues. Such signal inhibitors are described in Powis, G., and Kozikowski A., (1994 New Molecular Targets for Cancer Chemotherapy ed., Paul Workman and David Kerr, CRC press 1994, London.

Another group of signal transduction pathway inhibitors are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block ras activation in cells containing wild type mutant ras, thereby acting as antiproliferation agents. Ras oncogene inhibition is discussed in Scharovsky, O.G., Rozados, V.R., Gervasoni, S.I. Matar, P. (2000), Journal of Biomedical Science. 7(4 292-8; Ashby, M.N. (1998), Current Opinion in Lipidology. 9 (2) 99 - 102; and Bennett, C.F. and Cowsert, L.M. BioChim. Biophys. Acta, (1999) 1489(1):19-30.

As mentioned above, antibody antagonists to receptor kinase ligand binding may also serve as signal transduction inhibitors. This group of signal transduction pathway inhibitors includes the use of humanized antibodies to the extracellular ligand binding domain of receptor tyrosine kinases. For example Imclone C225 EGFR specific antibody (see Green, M.C. et al, Monoclonal Antibody Therapy for Solid Tumors, Cancer Treat. Rev., (2000), 26(4, 269-286); Herceptin® erbB2 antibody (see Tyrosine Kinase Signalling in Breast cancer:erbB Family Receptor Tyrosine Kniases, Breast cancer Res., 2000, 2(3), 176-183); and 2CB VEGFR2 specific antibody (see Brekken, R.A. et al, Selective Inhibition of VEGFR2 Activity by a monoclonal Anti-VEGF antibody blocks tumor growth in mice, Cancer Res. (2000) 60, 5117-5124.

Non-receptor kinase angiogenesis inhibitors may also find use in the present invention. Inhibitors of angiogenesis related VEGFR and TIE2 are discussed above in regard to signal transduction inhibitors (both receptors are receptor tyrosine kinases). Angiogenesis in general is linked to erbB2/EGFR signaling since inhibitors of erbB2 and EGFR have been shown to inhibit angiogenesis, primarily VEGF expression. Thus, the combination of an erbB2/EGFR inhibitor with an inhibitor of angiogenesis makes sense. Accordingly, non-receptor tyrosine kinase inhibitors may be used in combination with the EGFR/erbB2 inhibitors of the present invention. For example, anti-VEGF antibodies, which do not recognize VEGFR (the receptor tyrosine kinase), but bind to the ligand; small molecule inhibitors of integrin ($alpha_v$ $beta_3$) that will inhibit angiogenesis; endostatin and angiostatin (non-RTK) may also prove useful in combination with the disclosed erb family inhibitors. (See Bruns CJ et al (2000), Cancer Res., 60: 2926-2935; Schreiber AB, Winkler ME, and

Derynck R. (1986), Science, 232: 1250-1253; Yen L et al. (2000), Oncogene 19: 3460-3469).

Agents used in immunotherapeutic regimens may also be useful in combination with the compounds of formula (I). There are a number of immunologic strategies to generate an immune response against erbB2 or EGFR. These strategies are generally in the realm of tumor vaccinations. The efficacy of immunologic approaches may be greatly enhanced through combined inhibition of erbB2/EGFR signaling pathways using a small molecule inhibitor. Discussion of the immunologic/tumor vaccine approach against erbB2/EGFR are found in Reilly RT et al. (2000), Cancer Res. 60: 3569-3576; and Chen Y, Hu D, Eling DJ, Robbins J, and Kipps TJ. (1998), Cancer Res. 58: 1965-1971.

Agents used in proapoptotic regimens (e.g., bcl-2 antisense oligonucleotides) may also be used in the combination of the present invention. Members of the Bcl-2 family of proteins block apoptosis. Upregulation of bcl-2 has therefore been linked to chemoresistance. Studies have shown that the epidermal growth factor (EGF) stimulates anti-apoptotic members of the bcl-2 family (i.e., mcl-1). Therefore, strategies designed to downregulate the expression of bcl-2 in tumors have demonstrated clinical benefit and are now in Phase II/III trials, namely Genta's G3139 bcl-2 antisense oligonucleotide. Such proapoptotic strategies using the antisense oligonucleotide strategy for bcl-2 are discussed in Water JS et al. (2000), J. Clin. Oncol. 18: 1812-1823; and Kitada S et al. (1994, Antisense Res. Dev. 4: 71-79.

Cell cycle signalling inhibitors inhibit molecules involved in the control of the cell cycle. A family of protein kinases called cyclin dependent kinases CDKs) and their interaction with a family of proteins termed cyclins controls progression through the eukaryotic cell cycle. The coordinate activation and inactivation of different cyclin/CDK complexes is necessary for normal progression through the cell cycle. Several inhibitors of cell cycle signalling are under development. For instance, examples of cyclin dependent kinases, including CDK2, CDK4, and CDK6 and inhibitors for the same are described in, for instance, Rosania et al, Exp. Opin. Ther. Patents (2000) 10(2):215-230.

In one embodiment, methods are provided for administering a HER2 inhibitor to a patient in need thereof comprising:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, administering to said patient a HER2 inhibitor.

In one embodiment, methods are provided for prescribing an HER2 inhibitor to a patient in need thereof comprising:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, prescribing to said patient a HER2 inhibitor.

In one embodiment, methods are provided for a treating cancer in a patient in need thereof comprises:

determining whether said patient has the 936C>T genotype at the rs3025039 reference single nucleotide polymorphism in VEGFA; and
if said patient has the 936C>T genotype at the rs3025039 reference single nucleotide polymorphism in VEGFA, administering to said patient a HER2 inhibitor.

In one embodiment, methods are provided for treating cancer in a patient in need thereof, the patient having been previously genotyped as having the 936C>T genotyped at the rs3025039 single nucleotide polymorphism in VEGFA, comprising administering to the patient a HER2 inhibitor. In a further embodiment the cancer is metastatic breast cancer. In yet a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

Another embodiment is a method of treating cancer in a patient in need thereof comprising: administering to the patient a HER2 inhibitor; and then determining whether said patient has the 936C>T genotype at the rs3025039 reference single nucleotide polymorphism in VEGFA. In a further embodiment is a method of treating cancer in a patient in need thereof comprising: administering to the patient a first HER2 inhibitor; and then determining whether said patient has the 936C>T genotype at the rs3025039 reference single nucleotide polymorphism in VEGFA, and then treating with a second HER2 inhibitor if the 936C>T genotype a the rs3025039 reference single polymorphism in VEGFA is found.

The methods of the present invention include testing a patient for the 936C>T genotype at the rs3025039 reference single nucleotide polymorphism in VEGFA. The methods may also include, but are not limited to, testing a patient for a genotype at least one single nucleotide polymorphism that is correlated with the 936C>T genotype at the

rs3025039 reference single nucleotide polymorphism in VEGFA.

In one embodiment is lapatinib for use in the treatment of cancer in a human classified as a responder, wherein a responder is characterized by the presence of a 936C>T genotyped at the rs3025039 reference single nucleotide polymorphism in VEGFA. In another embodiment is lapatinib for use in the treatment of cancer in a human classified as a responder to lapatinib, wherein a responder is characterized by the presence of a 936C>T genotyped at the rs3025039 reference single nucleotide polymorphism in VEGFA. In a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

In another embodiment is the use of lapatinib in the manufacture of a medicament for the treatment of cancer in a human classified as a responder to lapatinib, wherein a responder is characterized by the presence of a 936C>T genotype at the rs3025039 reference single nucleotide polymorphism in VEGFA.

In a different embodiment is the use of lapatinib characterized in that it is for the manufacture of a medicament for the treatment of cancer in a human classified as a responder to lapatinib, wherein a responder is characterized by the presence of a 936C>T genotype at the rs3025039 reference single nucleotide polymorphism in VEGFA.

In embodiments of the methods or uses herein comprising determining the presence of a 936C>T genotype at the rs3025039 reference single nucleotide polymorphism in VEGFA, or in methods or uses herein in patients or humans determined to have the the presence of a 936C>T genotype at the rs3025039 reference single nucleotide polymorphism in VEGFA, the methods further comprise observing or determining or monitoring an improvement in overall survival after treatment with or administration of lapatinib.

In some aspects the cancer is breast cancer. The cancer can be metastatic breast cancer, for example in each of the embodiments of the invention herein for the use of lapatinib. Cancer is selected from the group consisting of: colon cancer, breast cancer, metastatic breast cancer, renal cell carcinoma, melanoma, lung cancer including non-small cell lung cancer and adenocarcinoma, gastric cancer, colorectal cancer, neuroendocrine cancer, thyroid cancer, head and neck cancer, brain cancer, cervical cancer, bladder cancer, esophageal cancer, pancreatic cancer, prostate cancer, mesothelioma, liver-hepatobiliary cancer, multiple myeloma, leukemia, thyroid cancer including Hurthle cell, muscle sarcoma (leiomyosarcoma) and bone sarcoma (chonrosarcoma).

In one embodiment the HER2 inhibitor is a dual target inhibitor HER2/EGFR inhibitor.

In one aspect the HER2 inhibitor comprises a compound of Formula I:

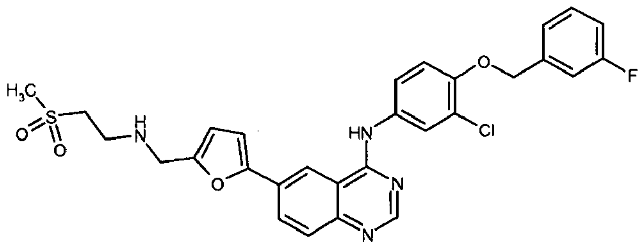

I

or a pharmaceutically acceptable salt or solvate thereof.

In another aspect, the HER2 inhibitor is a compound of Formula (I'):

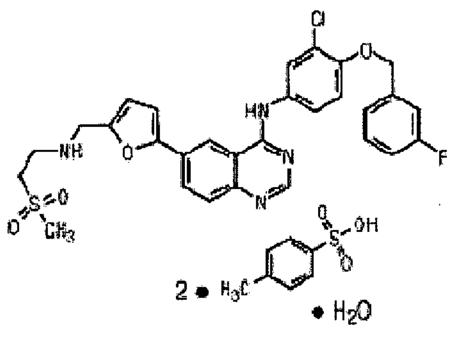

(I').

In one aspect, the HER2 inhibitor is a monoclonal antibody. The monoclonal antibody can be trastuzumab, pertuzumab or a combination of both. In one aspect the, HER2 inhibitor is administered as monotherapy. In one aspect the HER2 inhibitor is lapatinib or a pharmaceutically acceptable salt thereof and is administered in combination with

capecitabine and/or letrozole. In another aspect the HER2 inhibitor is lapatinib or a pharmaceutically acceptable salt thereof and is administered in combination with capecitabine and/or letrozole and/or trastuzumab.

Methods are also provided for treating a patient with cancer further comprising detecting whether said patient has a polymorphism in VEGFR2 18487A>T. This polymorphism is non-synonymous, Q472H, coding for an amino acid change at position 472 from Glutamine (Q) to Histidine (H). In one aspect the methods comprise treating said patient with lapatinib and trastuzumab if said patient has at least one single nucleotide polymorphism that correlates with VEGFR2 18487A>T.

Methods are also provided for treating cancer in a patient in need thereof comprising:

determining whether said patient has a polymorphism VEGFR2 18487A>T; and
if said patient has a polymorphism VEGFR2 18487A>T, administering to said patient lapatinib and trastuzumab.

In some embodiments, methods are provided for treating cancer in a patient in need thereof, the patient having been previously genotyped as having the 18487A>T genotype at the rs1870377 single nucleotide polymorphism in VEGFR2, comprising administering to the patient a HER2 inhibitor. In a further embodiment the cancer is metastatic breast cancer. In yet a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

Another embodiment is a method of treating cancer in a patient in need thereof comprising: administering to the patient a HER2 inhibitor; and then determining whether said patient has a 18487A>T genotype at the rs1870377 reference single nucleotide polymorphism in VEGFR2. In a further embodiment is a method of treating cancer in a patient in need thereof comprising: administering to the patient a first HER2 inhibitor; and then determining whether said patient has the 18487A>T genotype at the rs1870377 reference single nucleotide polymorphism in VEGFR2, and then treating with at least one additional HER2 inhibitor if the 18487A>T genotype a the rs1870377 reference single polymorphism in VEGFR2 is found. In a further embodiment, the first HER2 inhibitor is trastuzumab. In a further embodiment, the at least one additional HER2 inhibitor is lapatinib. In a further embodiment, the method comprises treating with at least one additional HER2 inhibitor that is lapatinib and further comprises treating with trastuzumab.

The methods of the present invention include testing a patient for the 18487A>T genotype at the rs1870377 reference single nucleotide polymorphism in VEGFR2. The methods may also include, but are not limited to, testing a patient for a genotype at least one single nucleotide polymorphism that is correlated with the 18487A>T genotype at the rs1870377 reference single nucleotide polymorphism in VEGFR2.
In one embodiment is lapatinib for use in the treatment of cancer in a human classified as a responder, wherein a responder is characterized by the presence of a 18487A>T genotype at the rs1870377 reference single nucleotide polymorphism in VEGFR2. In another embodiment is lapatinib for use in the treatment of cancer in a human classified as a responder to lapatinib, wherein a responder is characterized by the presence of a 18487A>T genotype at the rs1870377 reference single nucleotide polymorphism in VEGFR2. In a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

In another embodiment is the use of lapatinib in the manufacture of a medicament for the treatment of cancer in a human classified as a responder to lapatinib, wherein a responder is characterized by the presence of a 18487A>T genotype at the rs1870377 reference single nucleotide polymorphism in VEGFR2.

In a different embodiment is the use of lapatinib characterized in that it is for the manufacture of a medicament for the treatment of cancer in a human classified as a responder to lapatinib, wherein a responder is characterized by the presence of a 18487A>T genotype at the rs1870377 reference single nucleotide polymorphism in VEGFR2.

In embodiments of the methods or uses herein comprising determining the presence of a 936C>T genotype at the rs3025039 reference single nucleotide polymorphism in VEGFA, or in methods or uses herein in patients or humans determined to have the the presence of a 936C>T genotype at the rs3025039 reference single nucleotide polymorphism in VEGFA, the methods further comprise observing or determining or monitoring an improvement in overall survival after treatment with or administration of lapatinib and/or after treatment with or administration of lapatinib and trastuzumab.

In other embodiments of the invention that comprise determining or detecting 18487A>T VEGFR2 genotype (or the 18487A>T genotype at the rs1870377 reference single nucleotide polymorphism in VEGFR2), the 18487A>T VEGFR2 genotype is detected and/or determined using methods of detecting the nonsynonymous mutation in the gene product of the VEGFR2 gene, i.e. the Q472H mutation in the VEGFR2 protein is detected and or determined. Such methods of detection using protein are well known in the art.

Methods are also provided for treating a patient with cancer comprising:

determining whether said patient has at least one polymorphism selected from: IGF1R (rs2037448) 229741A>G and IGF1R (rs7181022) 28322 C>T; and
if said patient does not have a polymorphism selected from IGF1R (rs2037448) 229741A>G and IGF1R (rs7181022) 28322 C>T, administering to said patient lapatinib and trastuzumab.

In another embodiment is lapatinib for use in the treatment of cancer in a human classified as a responder, for example a human classified as a responder to lapatinib, not having a polymorphism selected from from IGF1R (rs2037448) 229741A>G and IGF1R (rs7181022) 28322 C>T. In a further embodiment is the use of trastuzumab in addition to lapatinib for use in the treatment of cancer in a human classified as a responder, for example a human classified as a responder to lapatinib, not having a polymorphism selected from from IGF1R (rs2037448) 229741A>G and IGF1R (rs7181022) 28322 C>T. In another embodiment is the use of lapatinib or lapatinib and trastuzumab for use in the manufacture of a medicament for the treatment of cancer for in a human classified as a responder, for example a human classified as a responder to lapatinib, not having a polymorphism selected from IGF1R (rs2037448) 229741A>G and IGF1R (rs7181022) 28322 C>T. In another embodiment is the use of lapatinib or lapatinib and trastuzumab characterized in that said lapatinib, or said lapatinib and trastuzumab, is for the manufacture of a medicament for the treatment of cancer for in a human classified as a responder, for example a human classified as a responder to lapatinib, not having a polymorphism selected from IGF1R (rs2037448) 229741A>G and IGF1R (rs7181022) 28322 C>T. In further embodiments for the treatment with or use of lapatinib, or lapatinib and trastuzumab in a human or patient not having a polymorphism selected from IGF1R (rs2037448) 229741A>G and IGF1R (rs7181022) 28322 C>T, the cancer is metastatic breast cancer.

In another embodiment methods are provided for treating cancer in a patient in need thereof comprises:

determining whether said patient has at least one polymorphism selected from:

VEGFR2 18487A>T and the 936C>T genotype at the rs3025039 reference single nucleotide polymorphism in VEGFA; and
if said patient at least one polymorphism selected from: VEGFR2 18487A>T and the 936C>T genotype at the rs3025039 reference single nucleotide polymorphism in VEGFA, administering to said patient a tyrosine kinase inhibitor.

Methods are also provided for treating cancer in a patient in need thereof comprising:

determining whether said patient has a polymorphism VEGFR2 18487A>T; and
if said patient has a polymorphism VEGFR2 18487A>T, administering to said patient lapatinib and trastuzumab.

In some embodiments, methods are provided for treating cancer in a patient in need thereof, the patient having been previously genotyped as having the tag SNP rs2307420 in the NR1I3, comprising administering to the patient a HER2 inhibitor. In a further embodiment the cancer is metastatic breast cancer. In yet a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

Another embodiment is a method of treating cancer in a patient in need thereof comprising: administering to the patient a HER2 inhibitor; and then determining whether said patient has tag SNP rs2307420 in the NR1I3 gene. In a further embodiment is a method of treating cancer in a patient in need thereof comprising: administering to the patient a first HER2 inhibitor; and then determining whether said patient has the tag SNP rs2307420 in NR1I3, and then treating with at least one additional HER2 inhibitor if the tag SNP rs2307420 in NR1I3 is found. In a further embodiment, the first HER2 inhibitor is trastuzumab. In a further embodiment, the at least one additional HER2 inhibitor is lapatinib. In a further embodiment, the method comprises treating with at least one additional HER2 inhibitor that is lapatinib and further comprises treating with trastuzumab.

The methods of the present invention include testing a patient for the tag SNP rs2307420 in NR1I3. The methods may also include, but are not limited to, testing a patient for a genotype having at least one single nucleotide polymorphism that is correlated with the tag SNP rs2307420 in NR1I3.

In one embodiment is lapatinib for use in the treatment of cancer in a human classified as a responder, wherein a responder is characterized by the presence of a tag SNP rs2307420 in NR1I3. In another embodiment is lapatinib for use in the treatment of cancer in a human classified as a responder to lapatinib, wherein a responder is characterized by the presence of a tag SNP rs2307420 in NR1I3. In a further embodiment, the cancer is metastatic breast cancer in a patient in further need of treatment following administration or treatment with a HER2 inhibitor. In further embodiments the further need of treatment follows administration of a HER2 inhibitor that is a monoclonal antibody, including but not limited to trastuzumab.

In another embodiment is the use of lapatinib in the manufacture of a medicament for the treatment of cancer in a human classified as a responder to lapatinib, wherein a responder is characterized by the presence of a tag SNP rs2307420 in NR1I3.

In a different embodiment is the use of lapatinib characterized in that it is for the manufacture of a medicament for the treatment of cancer in a human classified as a responder to lapatinib, wherein a responder is characterized by the presence of a tag SNP rs2307420 in NR1I3.

In embodiments herein of methods comprising detecting or determining the tag SNP rs2307420 in NR1I3, or in treating patients having been determined to have the tag SNP rs2307420 in NR1I3, the methods further comprise monitoring or determining or observing an improvement in progression free survival after treatment with lapatinib and or treatment with lapatanib and trastuzumab.

Also provided are biomarkers for use in therapy or treatment of cancer. In one embodiment, the biomarker for use in therapy or treatment of cancer is selected from the group consisting of: the presence of a 936C>T genotype at the rs3025039 reference single nucleotide polymorphism in VEGFA, the presence of a polymorphism VEGFR2 18487A>T, the presence of a Q472H mutation in the VEGFR2 protein, the presence of a tag SNP rs2307420 in NR1I3, the absence of a IGF1R (rs2037448) 229741A>G polymorphism, and the absence of a IGF1R (rs7181022) 28322 C>T polymorphism. In further embodiments, the biomarker is for use in therapy or treatment of metastatic breast cancer. In further embodiments, the biomarker is for use in lapatanib therapy or lapatinib treatment of metastatic breast cancer. In further embodiments the biomarker is a combination of two, three, four, five of the polymorphisms of the recited group.

In further embodiments, the methods of the invention further comprise administering at least one additional neo-plastic agent to said patient.

The invention is further described by the following non-limiting examples.

## EXAMPLES

### Example 1

Lapatinib combinations are effective therapy in treating patients with metastatic breast cancer (MBC) whose tumors overexpress HER2. Consistent with HER2/EGFR and other tyrosine kinase inhibitor (TKI) therapies, patient response is variable, suggestive of additional determinants of sensitivity and resistance. Host, germline genetic variation has been associated with TKIs used to treat other cancers. This exploratory pharmacogenetic study sought to identify germline genetic variants that associate with differential lapatinib treatment outcomes in HER2+ MBC patients.

### Experimental methods

Objective: Identify germline genetic variants that predict differential patient response to lapatinib treatment, in HER2-positive women, measured by primary endpoints of progression free survival (PFS) and overall survival (OS) in the following patient populations:

- Clinical Trial I: lapatinib monotherapy study in HER2+ MBC patients with recurrent brain metastases following trastuzumab based systemic therapy and cranial radiotherapy (n=120).
- Clinical Trial II: lapatinib plus trastuzumab (n=92) and lapatinib monotherapy (n=103) study in,HER2+ MBC patients with disease progression following trastuzumab based therapy.

Fifty five single nucleotide polymorphisms (SNPs) with functional consequence in 24 candidate genes were evaluated. The assay platform used to genotype these SNPs was the Illumina Human 1M-Duo BeadChip. Candidate genes for this experiment are listed below.

| Gene | Category | Function |
|---|---|---|
| ABCB1 | ADME | (P-GP) lapatinib is substrate |
| ABCG2 | ADME | (BCRP) lapatinib is substrate |
| AKT1 | Pathway | Involved in anti-apoptosis and in pro-cell proliferation |
| CXCL12 | Pathway, Alternative angiogenesis | aka SDF1, regulates endothelial progenitor cell in angiogenesis |
| CYP3A4 | ADME | Lapatinib is substrate & inhibitor |

(continued)

| Gene | Category | Function |
|---|---|---|
| CYP3A5 | ADME | CYP3A4 'analogue' |
| EGF | Pathway | Lapatinib and trastuzumab target. Forms heterodimer with ErbB2. |
| EGFR | Pathway | Lapatinib and trastuzumab target. Forms heterodimer with ErbB2. |
| ErbB2 | Pathway | Lapatinib and trastuzumab target. Forms heterodimers with EGFR/ErbB1, ErbB3 and IGF1R |
| ErbB3 | Pathway | (Her3) Forms heterodimer with ErbB2 |
| FGF2 | Pathway, Alternative angiogenesis | FGFR2 ligand |
| FGFR2 | Pathway, Alternative angiogenesis | Regulator of angiogenesis. Multiple replicated associations with BC risk. |
| FLT4 | angiogenesis | (VEGFR3) |
| HIF1A | Pathway, Alternative angiogenesis | Ligand for MEK, Regulator of angiogenesis. A588T polymorphism associated with pazopanib response in mRCC. |
| IGF1 | Pathway, Alternative cell signaling | Regulator of insulin homeostasis. Associated with BC risk. Associated with cetuximab response in mCRC. IGFR1/EGFR heterodimers activated by TGF. IGF1R inhibition improves trastuzumab cell line response. |
| IGF1R | Pathway, Alternative cell signaling | Forms heterodimer with ErbB2. Associated with EGFR and HER2 inhibitor resistance. Associated with cetuximab response in mCRC. |
| IGFBP3 | Pathway, Alternative cell signaling | Sequesters circulating IGF1. High levels appear protective of cancer. |
| IL8 | Pathway, Alternative angiogenesis | Cytokine regulator of angiogenesis. High serum IL8 associated with poor VEGFR inhibitor response in mRCC |
| IL8RB | Pathway, Alternative angiogenesis | (CXCR2) receptor for IL8 |
| KDR | angiogenesis. | (VEGFR2) Polymorphism associated with pazopanib response in mRCC |
| NR1I2 | ADME | (PXR) regulates CYP3A4 expression. Polymorphism associated with pazopanib response in mRCC |
| NR1I3 | ADME | (RXR) regulates CYP3A4 expression. Polymorphism associated with sunitinib response in mRCC |
| TGFa | Pathway, Alternative cell signaling | EGFR ligand. High serum TGFa associated with poor lapatinib, gefitinib and trastuzumab response. |
| VEGFA | angiogenesis | VEGRF2 ligand. Polymorphism associated with pazopanib response in mRCC |

Statistical analysis of genetic associations were evaluated using an additive test. For any significant marker identified from the additive test, specific contrasts of interest between different genotypes will be explored to determine risk genotype(s). Cox proportional hazards model with Firth method. Any covariate significant at $p \leq 0.05$ in the multivariate Cox model will be included.

**Results:**

Fifty five single nucleotide polymorphisms (SNPs) with functional consequence in 24 candidate genes were evaluated in a subset of MBC patients participating in two clinical trials: Trial I: a lapatinib monotherapy study in HER2+ MBC patients with recurrent brain metastases following trastuzumab and cranial radiotherapy (n=120) and Trial II: lapatinib plus trastuzumab (n=92) and lapatinib monotherapy (n=103) study in HER2+ MBC patients with disease progression following trastuzumab. Testing for associations of SNPs with progression free survival (PFS) and overall survival

(OS) during lapatinib treatment was performed using Cox proportional hazards methods, with covariate adjustment. Markers were considered to be significantly associated if they achieved a predefined multiple testing threshold of p<0.0003.

No SNPs were statistically significantly associated with progression-free survival (PFS) in either study.

A SNP in VEGFA (rs3025039, 936C>T) was statistically significantly associated with overall survival (OS) (p=0.0002), with improved OS for T allele carriers and an allelic hazard ratio of 0.21 (0.08-0.52) in Trial I, but this association was not seen in Trial II. This SNP is located in the 3' UTR gene region, modulates serum VEGFA levels and is associated with breast cancer risk [Krippl et al., 2003, Int J Cancer 106: 468; Kataoka et al., 2006, Cancer Epidemiol Biomarkers Prev, 15:1148].

A SNP in VEGFR2 (rs1870377, 18487A>T, Q472H, NP_0022441) was statistically significantly associated with overall survival (OS) in Trial II (p=0.0004). Whilst this SNP did not achieve the highly conservative Bonferroni threshold (p<0.0003) used in this analysis, it was considered nominally significant. VEGFR2 (rs1870377, 18487A>T, Q472H) had an allelic hazard ratio of 0.47 (0.3-0.73) in Trial II with T allele carriers showing improved OS, but this association was not seen in Trial I. VEGFR2 18487A>T is non-synonymous (472Q>H) and has been reported to modulate VEGF binding to VEGFR2, with the T allele increasing receptor function (VEGF binding and VEGFR2 receptor phosphorylation.

Two SNPs in IGFR1 (rs2037448, 229741A>G and rs7181022, 28322C>T, NP_000866.1) were nominally associated with overall survival (OS) in both Trial I and II (p<0.05). These SNPs have low linkage disequilibrium correlation with each other and were combined for analysis with patients categorised as heterozygous for the minor allele for either rs2037448-T or rs7181022-G. Carriage of either specified IGFR1 minor allele resulted in a patient group who were statistically significantly associated with poorer overall survival (OS) than wild type (p=0.00028), with an allelic hazard ratio of 5.0 (2.2-10.0) in Trial II. A similar trend for association was observed in Trial I, but this trial did not achieve Bonferroni threshold for significant association (p=0.075). The function of these SNPs has not been identified.

**Conclusions:** A germline variant in VEGFA may be associated with survival outcome for lapatinib in MBC patients with brain metastases. This may represent activation of VEGF angiogenic pathways to overcome HER2 inhibition in patients carrying the higher expression genotype. Nonimal associations with OS were observed for a SNP in VEGFR2 (rs1870377) and two SNPs in IGFR1 (rs2037448 and rs7181022).

**Further analysis of Trial I and Trial II**

Additional pharmacogenentic analysis was performed using the patients in Trial I and II (described above), using both candidate gene selection and using a genome wide association study. (GWAS, 1M Genome-Wide SNPs (Illumina human 1M duo)). Genotyping was conducted as described previously (Spraggs et al, 2011, J Clin Oncol 29: 667). Examined genes were divided into three tiers as follows:

**Tier I:** 7 SNPs previously associated with expression, alternative signaling or ADME in TKI treatment response, or breast cancer susceptibility

**Tier II:** 48 functional variants from 23 candidate genes selected from the following categories:

- Lapatinib ADME (ABCB1, ABCG2, CYP3A4, CYP3A5, NR1I2, NR1I3)
- Lapatinib pathway (EGFR, ERBB2, ERBB3, IGF1, IGF1R, IGFBP3, EGF, AKT1)
- Associated with TKI resistance (HIF1A, IL8, IL8RB, CXCL12, VEGFA, VEGFR2, VEGFR3)
- Breast cancer susceptibility (FGF2, FGFR2)

**Tier III:** 1472 Tag SNPs in the 23 candidate genes listed above, plus TGF$\alpha$ (no common functional variants identified for TGF$\alpha$)

Associations were evaluated using an additive genetic test, using a multivariate Cox proportional hazards model adjusting for significant clinical covariates and race/ethnicity difference. Firth and Genomic Control methods were used for candidate genes and GWAS respectively to control for false positive results. The primary endpoints was Progression Free Survival (PFS), where the secondary endpoint was Overall Survival (OS).

In addition to testing within each unique study-treatment arm, meta-analysis, conducted by inverse variance method, was used to combine results. Variants were assigned to tiers for analysis, with different association significance thresholds and these were specified prior to analysis to adjust for number of SNP tests, but not for number of endpoints and groups, as follows:

- Candidate genes:

| Tier | # SNPs | Alpha Spend | Threshold for Significance |
|---|---|---|---|
| I | 7 | 0.03 | 0.004 |
| II | 48 | 0.015 | 0.0003 |
| III | 1472 | 0.005 | $3.40\times10^{-6}$ |
| Total | 1515 | 0.05 | |

• GWAS SNPs: Bonferroni threshold for significance $p = 0.05 / 1M = 5\times10^{-8}$

**Results**

In meta-analysis, the NR1I3 (rs2307420) marker passed the p value threshold at $3.4 \times 10^{-6}$ for PFS. NR1I3 is a constitutive androdane receptor, and regulates CYP3A4 expression. The effect is driven by carriage of the low frequency G allele (n≤5). The rs2307420 is a tag SNP, which is not functional.

In Clinical Trial I (lapatanib monotherapy), the VEGFA (936C>T, rs3025039) marker passed the threshold at $p < 0.0003$ for OS. The effect was driven by the results analyzed from Clinical Trial I (monotherapy arm), but opposite effect were seen in Clinical Trial II (lapatinib plus trastuzumab arm). VEGFA 936C>T (rs3025039) is located in the 3'UTR and modulates VEGF expression. CC has higher VEGF expression than CT/TT (Formento et al, 2009, Pharmacogenomics, 10: 1277; Krippl et al, 2003, Int J Cancer 106: 468, Kataoka et al, 2006, Cancer Epidemiol Biomarker Prev, 15: 1148). Results are shown in the Figures.

There was also a marginal association (p=0.0005 for KDR/VEGFR2 in Clincal Trial II (lapatanib plus tratuzumab) for OS. Results are shown in Figure 4C.

GWAS analysis showed no strong signals ($p<5\times10^{-8}$) across the trials or study arms with common markers (MAF >5%).

## Claims

1. Lapatinib for use in the treatment of cancer in a human classified as a responder, wherein a responder is **characterized by** the presence of a 18487A>T genotype at the rs1870377 reference single nucleotide polymorphism in VEGFR2.

2. Lapatinib for use in the treatment of cancer in a human classified as a responder, wherein a responder is **characterized by** not having a polymorphism selected from IGF1R (rs2037448) 229741A>G and IGF1R (rs7181022) 28322 C>T.

3. A combination of lapatinib and trastuzumab, for use in the treatment of cancer in a human classified as a responder, wherein a responder is **characterized by** the presence of a 18487A>T genotype at the rs1870377 reference single nucleotide polymorphism in VEGFR2.

4. A combination of lapatinib and trastuzumab for use in the treatment of cancer in a human classified as a responder, wherein a responder is **characterized by** not having a polymorphism selected from IGF1R (rs2037448) 229741A>G and IGF1R (rs7181022) 28322 C>T.

5. Lapatinib for use as claimed in claims 1 or 2, or a combination for use as claimed in claims 3 or 4, wherein the cancer is selected from the group consisting of: colon cancer, breast cancer, metastatic breast cancer, renal cell carcinoma, melanoma, lung cancer including non-small cell lung cancer and adenocarcinoma, gastric cancer, colorectal cancer, neuroendocrine cancer, thyroid cancer, head and neck cancer, brain cancer, cervical cancer, bladder cancer, esophageal cancer, pancreatic cancer, prostate cancer, mesothelioma, liver-hepatobiliary cancer, multiple myeloma, leukemia, thyroid cancer including Hurthle cell, muscle sarcoma (leiomyosarcoma) and bone sarcoma (chondrosarcoma).

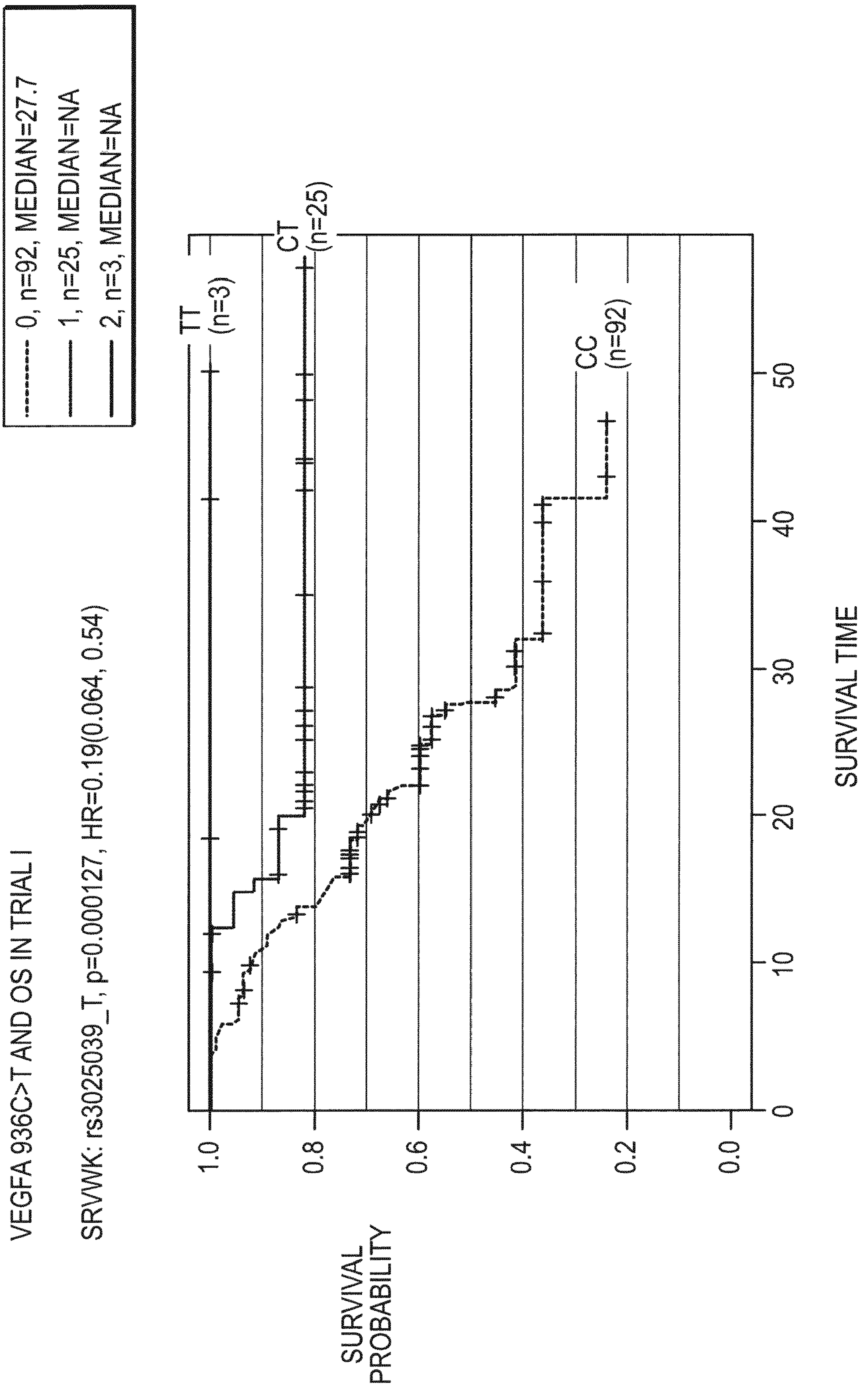
VEGFA 936C>T AND OS IN TRIAL I
SRVWK: rs3025039_T, p=0.0001270, HR=0.19(0.064, 0.54)
0, n=92, MEDIAN=27.7
1, n=25, MEDIAN=NA
2, n=3, MEDIAN=NA
SURVIVAL PROBABILITY
1.0
0.8
0.6
0.4
0.2
0.0
0
10
20
30
40
50
SURVIVAL TIME
TT (n=3)
CT (n=25)
CC (n=92)

*FIG. 1*

FIG. 2

VEGFR2 18487A>T (Q472H) and OS in Trial II

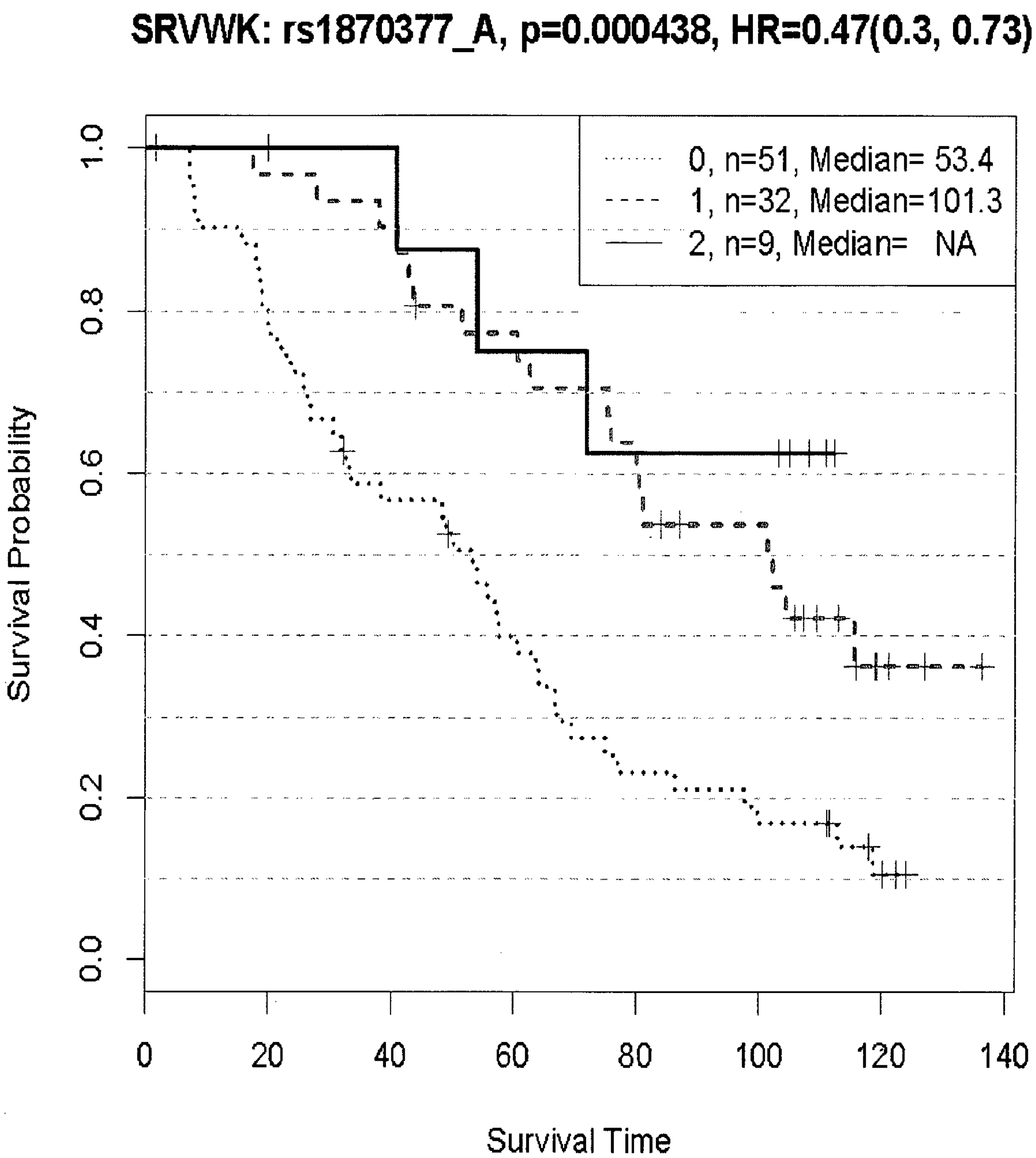

**FIG. 3**

IGFR1 rs2037448 & rs7181022 (tag SNPs, not functional) and OS in Trial II

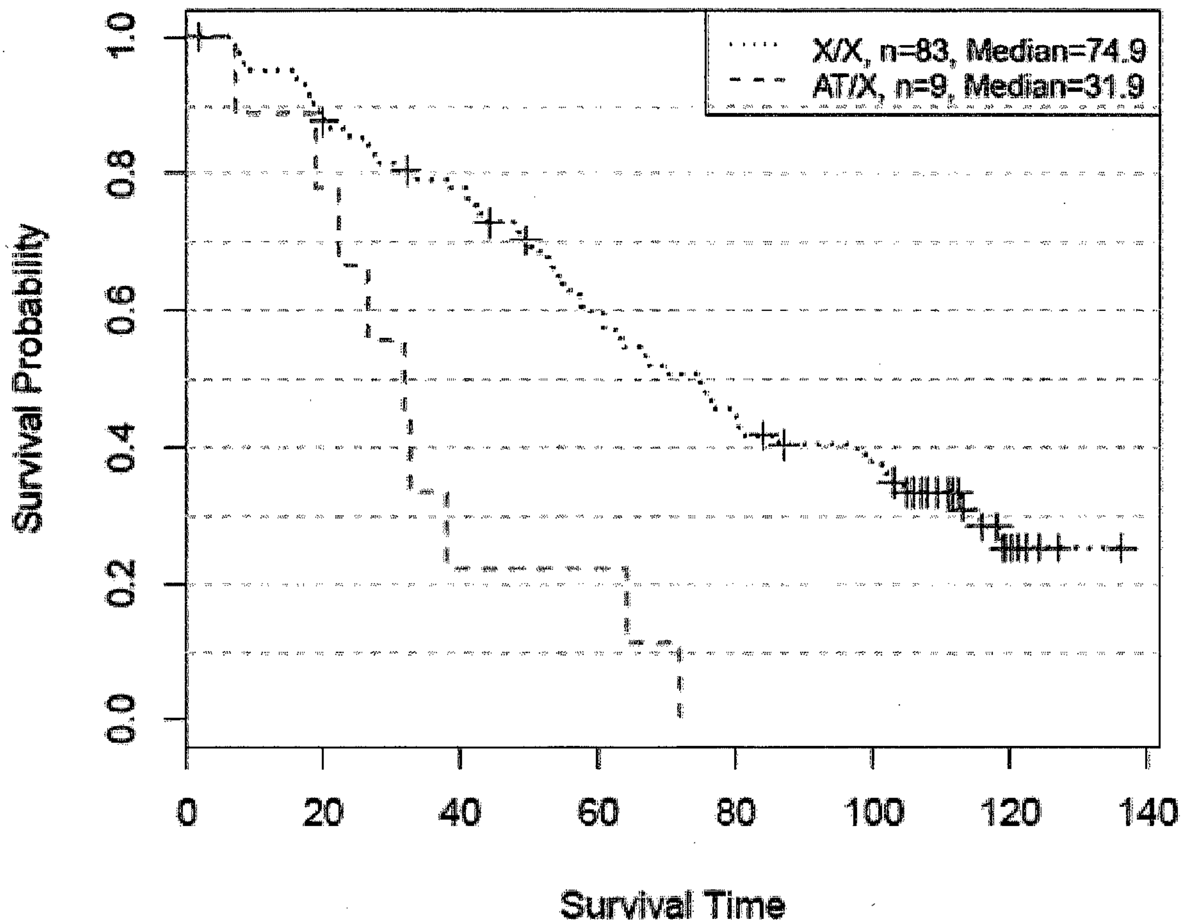

FIG. 4

## A) *NR1I3* (Tag SNP, rs2307420) and PFS

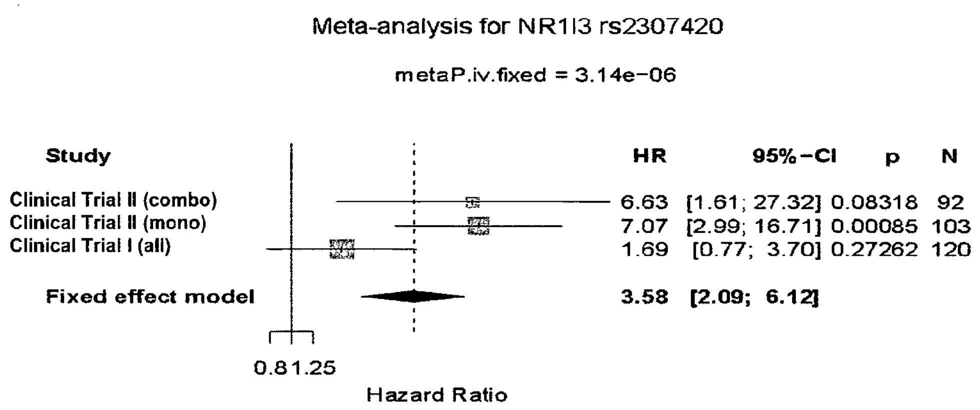


## B) *VEGFA* (936C>T, rs3025039) and OS

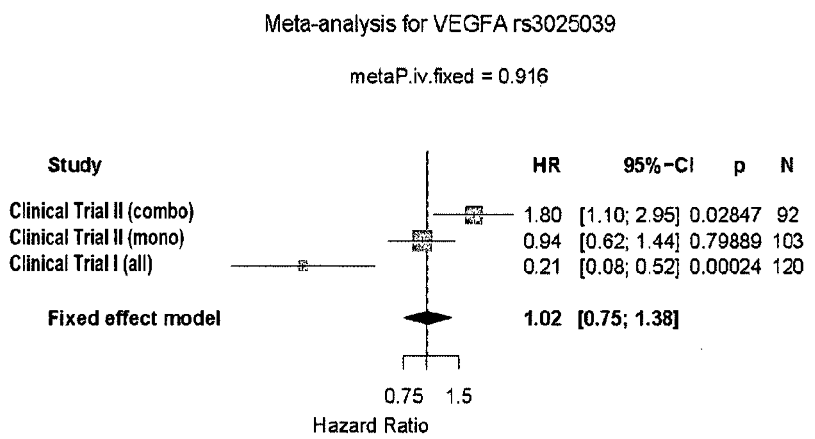


## C) *KDR/VEGFR2* (18487T>A, Q472H, rs1870377) and OS

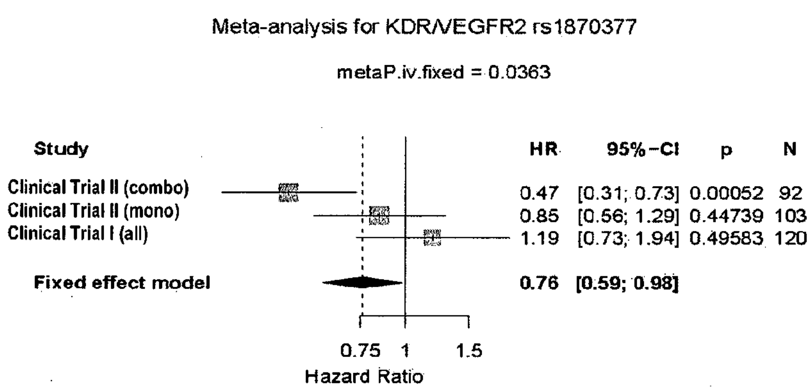

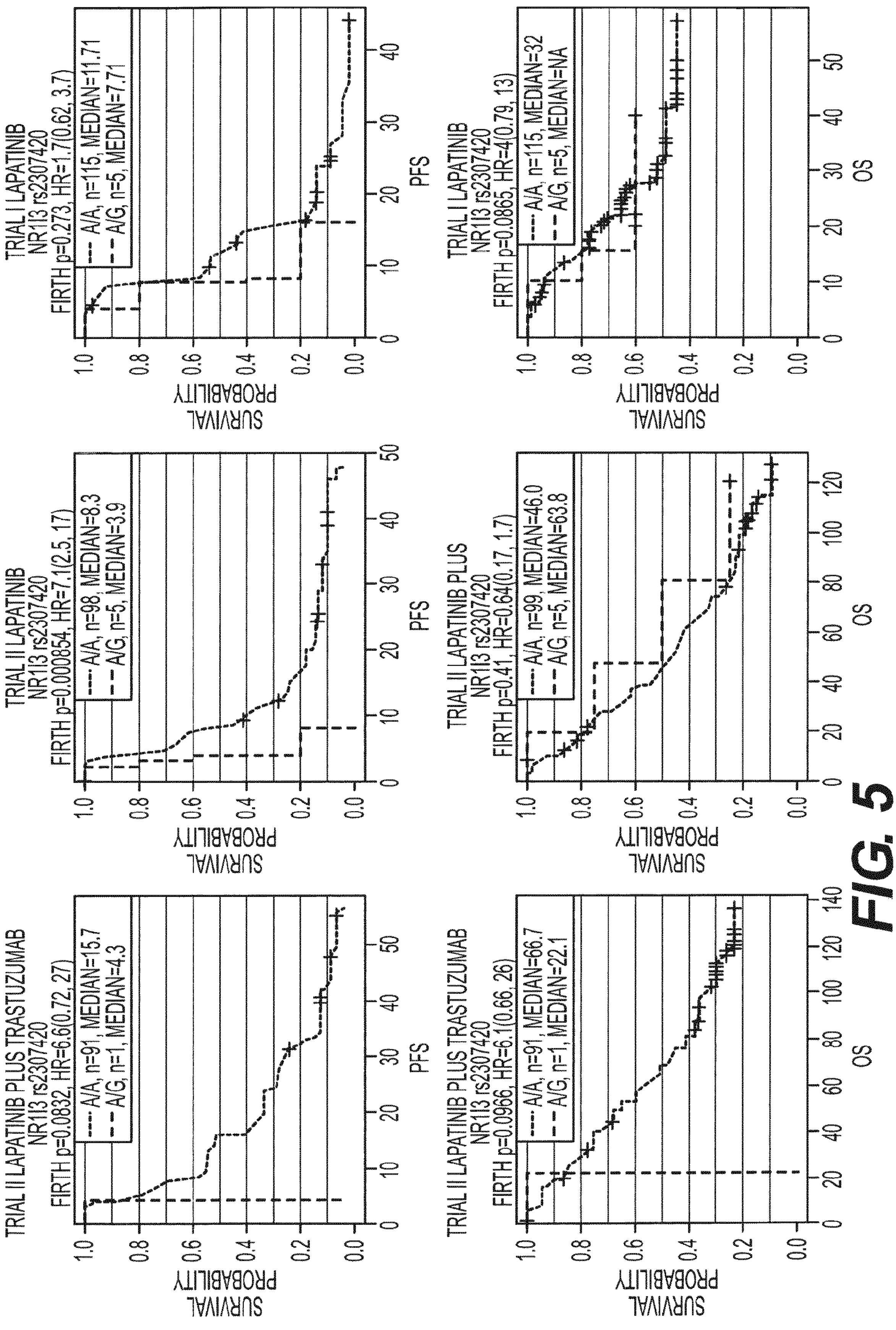
TRIAL II LAPATINIB PLUS TRASTUZUMAB
NR1I3 rs2307420
FIRTH p=0.0832, HR=6.6(0.72, 27)
A/A, n=91, MEDIAN=15.7
A/G, n=1, MEDIAN=4.3
SURVIVAL PROBABILITY
PFS
TRIAL II LAPATINIB
NR1I3 rs2307420
FIRTH p=0.000854, HR=7.1(2.5, 17)
A/A, n=98, MEDIAN=8.3
A/G, n=5, MEDIAN=3.9
SURVIVAL PROBABILITY
PFS
TRIAL I LAPATINIB
NR1I3 rs2307420
FIRTH p=0.273, HR=1.7(0.62, 3.7)
A/A, n=115, MEDIAN=11.71
A/G, n=5, MEDIAN=7.71
SURVIVAL PROBABILITY
PFS
TRIAL II LAPATINIB PLUS TRASTUZUMAB
NR1I3 rs2307420
FIRTH p=0.0966, HR=6.1(0.66, 26)
A/A, n=91, MEDIAN=66.7
A/G, n=1, MEDIAN=22.1
SURVIVAL PROBABILITY
OS
TRIAL II LAPATINIB PLUS
NR1I3 rs2307420
FIRTH p=0.41, HR=0.64(0.17, 1.7)
A/A, n=99, MEDIAN=46.0
A/G, n=5, MEDIAN=63.8
SURVIVAL PROBABILITY
OS
TRIAL I LAPATINIB
NR1I3 rs2307420
FIRTH p=0.0865, HR=4(0.79, 13)
A/A, n=115, MEDIAN=32
A/G, n=5, MEDIAN=NA
SURVIVAL PROBABILITY
OS

FIG. 5

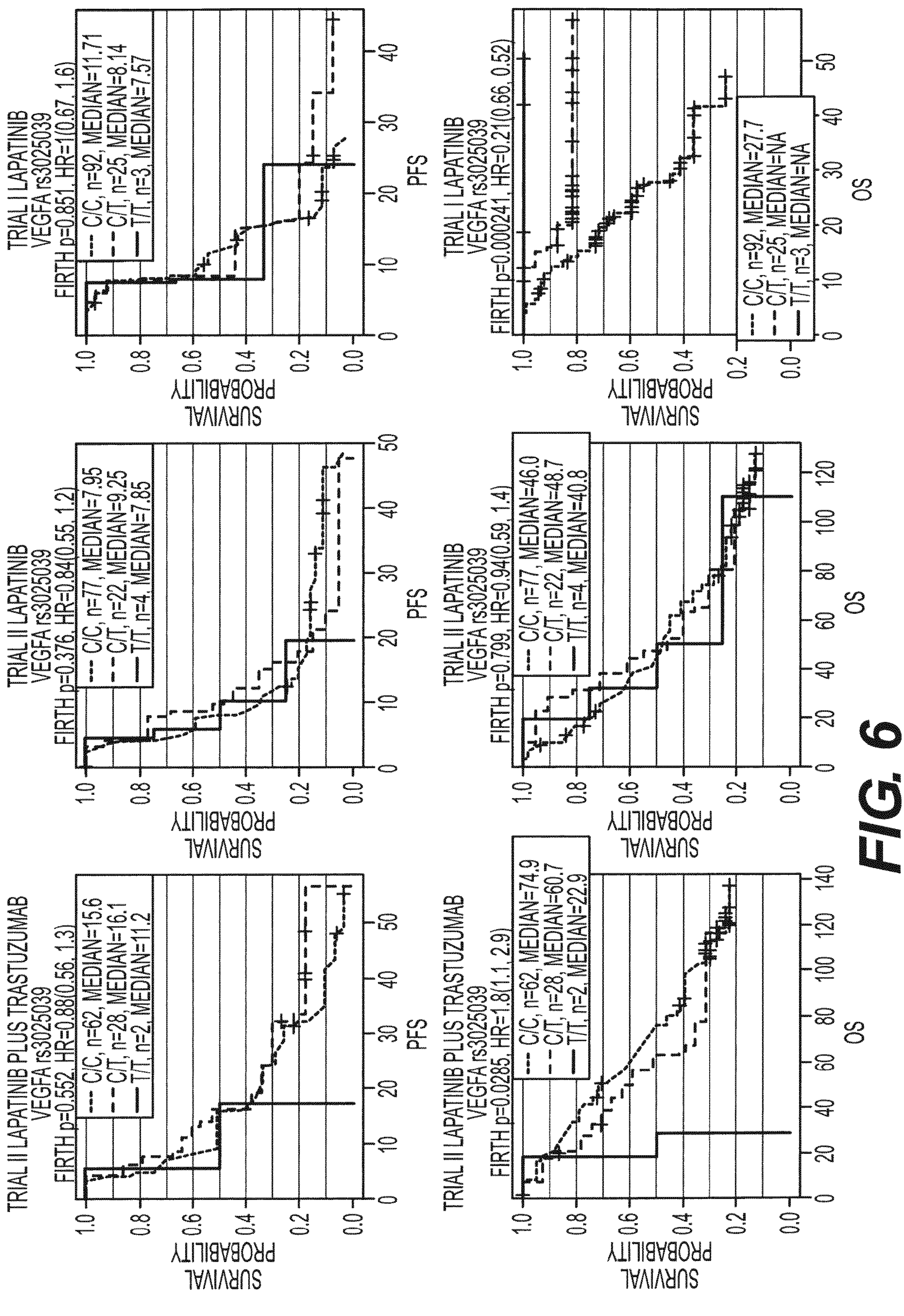
TRIAL II LAPATINIB PLUS TRASTUZUMAB
VEGFA rs3025039
FIRTH p=0.552, HR=0.88(0.56, 1.3)
C/C, n=62, MEDIAN=15.6
C/T, n=28, MEDIAN=16.1
T/T, n=2, MEDIAN=11.2
SURVIVAL PROBABILITY
PFS
TRIAL II LAPATINIB
VEGFA rs3025039
FIRTH p=0.376, HR=0.84(0.55, 1.2)
C/C, n=77, MEDIAN=7.95
C/T, n=22, MEDIAN=9.25
T/T, n=4, MEDIAN=7.85
SURVIVAL PROBABILITY
PFS
TRIAL I LAPATINIB
VEGFA rs3025039
FIRTH p=0.851, HR=1(0.67, 1.6)
C/C, n=92, MEDIAN=11.71
C/T, n=25, MEDIAN=8.14
T/T, n=3, MEDIAN=7.57
SURVIVAL PROBABILITY
PFS
TRIAL II LAPATINIB PLUS TRASTUZUMAB
VEGFA rs3025039
FIRTH p=0.0285, HR=1.8(1.1, 2.9)
C/C, n=62, MEDIAN=74.9
C/T, n=28, MEDIAN=60.7
T/T, n=2, MEDIAN=22.9
SURVIVAL PROBABILITY
OS
TRIAL II LAPATINIB
VEGFA rs3025039
FIRTH p=0.799, HR=0.94(0.59, 1.4)
C/C, n=77, MEDIAN=46.0
C/T, n=22, MEDIAN=48.7
T/T, n=4, MEDIAN=40.8
SURVIVAL PROBABILITY
OS
TRIAL I LAPATINIB
VEGFA rs3025039
FIRTH p=0.000241, HR=0.21(0.66, 0.52)
C/C, n=92, MEDIAN=27.7
C/T, n=25, MEDIAN=NA
T/T, n=3, MEDIAN=NA
SURVIVAL PROBABILITY
OS

FIG. 6

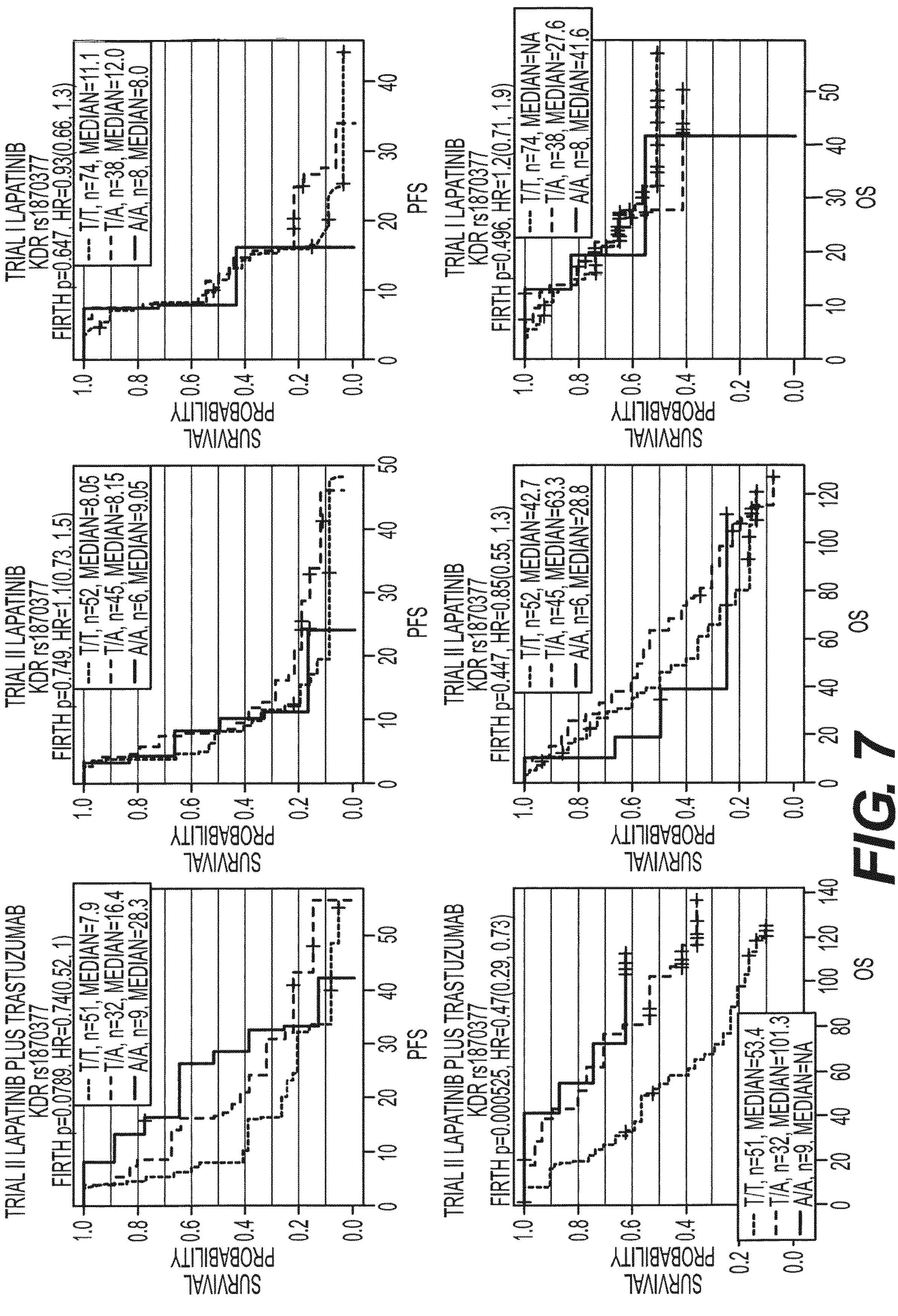
TRIAL II LAPATINIB PLUS TRASTUZUMAB
KDR rs1870377
FIRTH p=0.0789, HR=0.74(0.52, 1)
T/T, n=51, MEDIAN=7.9
T/A, n=32, MEDIAN=16.4
A/A, n=9, MEDIAN=28.3
SURVIVAL PROBABILITY
PFS
TRIAL II LAPATINIB
KDR rs1870377
FIRTH p=0.749, HR=1.1(0.73, 1.5)
T/T, n=52, MEDIAN=8.05
T/A, n=45, MEDIAN=8.15
A/A, n=6, MEDIAN=9.05
SURVIVAL PROBABILITY
PFS
TRIAL I LAPATINIB
KDR rs1870377
FIRTH p=0.647, HR=0.93(0.66, 1.3)
T/T, n=74, MEDIAN=11.1
T/A, n=38, MEDIAN=12.0
A/A, n=8, MEDIAN=8.0
SURVIVAL PROBABILITY
PFS
TRIAL II LAPATINIB PLUS TRASTUZUMAB
KDR rs1870377
FIRTH p=0.000525, HR=0.47(0.29, 0.73)
T/T, n=51, MEDIAN=53.4
T/A, n=32, MEDIAN=101.3
A/A, n=9, MEDIAN=NA
SURVIVAL PROBABILITY
OS
TRIAL II LAPATINIB
KDR rs1870377
FIRTH p=0.447, HR=0.85(0.55, 1.3)
T/T, n=52, MEDIAN=42.7
T/A, n=45, MEDIAN=63.3
A/A, n=6, MEDIAN=28.8
SURVIVAL PROBABILITY
OS
TRIAL I LAPATINIB
KDR rs1870377
FIRTH p=0.496, HR=1.2(0.71, 1.9)
T/T, n=74, MEDIAN=NA
T/A, n=38, MEDIAN=27.6
A/A, n=8, MEDIAN=41.6
SURVIVAL PROBABILITY
OS

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6391874 B
- US 6828320 B
- US 6727256 B
- US 6713485 B
- US 7157466 B
- WO 9740462 A
- US 5871918 A, Thorp
- WO 0100245 A
- WO 9935146 A
- WO 0202552 A
- US 5747498 A
- GB 9600961 W
- WO 9633980 A
- US 6063923 A
- US 5342947 A
- US 5559235 A
- US 5491237 A
- US 97721797 A
- US 5681835 A
- US 5877219 A
- US 6207716 B
- US 6268391 B


### Non-patent literature cited in the description


- **STEPHENS et al.** *Science,* 20 July 2001, vol. 293, 489-493
- **LINDER et al.** *Clinical Chemistry,* 1997, vol. 43, 254
- **MARSHALL.** *Nature Biotechnology,* 1997, vol. 15, 1249
- **SCHAFER et al.** *Nature Biotechnology,* 1998, vol. 16, 3
- **MAXAM ; GILBERT.** *Proc. Natl. Acad. Sci. USA,* 1977, vol. 74, 560
- **SANGER et al.** *Proc. Natl. Acad. Sci. USA,* 1977, vol. 74, 5463
- **SAMBROOK ; FRITSCH ; MANIATIS.** Molecular Cloning: A Laboratory Manual. Cold Spring Harbor Laboratory Press, 1989
- DNA Cloning: A Practical Approach. 1985, vol. I and II
- Oligonucleotide Synthesis. 1984
- Nucleic Acid Hybridization. 1985
- Transcription And Translation. 1984
- Animal Cell Culture. 1986
- Immobilized Cells And Enzymes. IRL Press, 1986
- **B. PERBAL.** *A Practical Guide To Molecular Cloning,* 1984
- Current Protocols in Molecular Biology. John Wiley & Sons, Inc, 1994
- **NIELSEN et al.** *Science,* 1991, vol. 254, 1497-1500
- **EGHOLM et al.** *J. Am. Chem. Soc.,* 1992, vol. 114, 1895-1897
- **JAMES et al.** *Protein Science,* 1994, vol. 3, 1347-1350
- **TATON et al.** *Science,* 2000, vol. 289, 1757-60
- **LOCKHART et al.** *Nature,* 2000, vol. 405, 827-836
- **GERHOLD et al.** *Trends in Biochemical Sciences,* 1999, vol. 24, 168-73
- **WALLACE, R. W.** *Molecular Medicine Today,* 1997, vol. 3, 384-89
- **BLANCHARD ; HOOD.** *Nature Biotechnology,* 1996, vol. 149, 1649
- **ROEST et al.** *Human Molecular- Genetics,* 1993, vol. 2, 1719-1721
- **VAN DER LUIT et al.** *Genomics,* 1994, vol. 20, 1-4
- **HOGERVORST et al.** *Nature Genetics,* 1995, vol. 10, 208-212
- **BS WEIR.** *Genetic Data Analysis II Sinauer Associates,* 1996
- *PCR Methods and Applications,* 1994, vol. 3, 268-71
- *Proc. Natl. Acad. Sci.,* 1998, vol. 85, 4397-4401
- **MARRAS et al.** *Genet Anal,* 1999, vol. 14, 151
- **GUSEV et al.** *Am J Pathol,* 2001, vol. 159, 63
- **LIEDER.** *Advance for Laboratory Managers,* 2000, vol. 70
- Cancer Principles and Practice f Oncology. Lippincott Williams & Wilkins Publishers, 15 February 2001
- **WANI et al.** *J. Am. Chem, Soc.,* 1971, vol. 93, 2325
- **SCHIFF et al.** *Proc. Natl, Acad, Sci. USA,* 1980, vol. 77, 1561-1565
- **SCHIFF et al.** *Nature,* 1979, vol. 277, 665-667

- **KUMAR.** *J. Biol, Chem,* 1981, vol. 256, 10435-10441
- New trends in Natural Products Chemistry. **D. G. I. KINGSTON et al.** Studies in Organic Chemistry. Elsevier, 1986, vol. 26, 219-235
- **MARKMAN et al.** *Yale Journal of Biology and Medicine,* 1991, vol. 64, 583
- **MCGUIRE et al.** *Ann. Intem, Med.,* 1989, vol. 111, 273
- **HOLMES et al.** *J. Nat. Cancer Inst.,* 1991, vol. 83, 1797
- **EINZIG.** *Proc. Am. Soc. Clin. Oncol.,* vol. 20, 46
- **FORASTIRE.** *Sem. Oncol.,* 1990, vol. 20, 56
- **WOO.** *Nature,* 1994, vol. 368, 750
- **IGNOFF, R.J.** *Cancer Chemotherapy Pocket Guide,* 1998
- **KEARNS, C.M.** *Seminars in Oncology,* 1995, vol. 3 (6), 16-23
- **KATH, JOHN C.** *Exp. Opin. Ther. Patents,* 2000, vol. 10 (6), 803-818
- **SHAWVER et al.** *DDT,* February 1997, vol. 2 (2
- Growth factor receptors as targets. **LOFTS, F. J. et al.** New Molecular Targets for Cancer Chemotherapy. CRC press, 1994
- **SINH, S. ; COREY, S.J.** *Journal of Hematotherapy and Stem Cell Research,* 1999, vol. 8 (5), 465-80
- **BOLEN, J.B. ; BRUGGE, J.S.** *Annual review of Immunology,* 1997, vol. 15, 371-404
- **SMITHGALL, T.E.** *Journal of Pharmacological and Toxicological Methods,* 1995, vol. 34 (3), 125-32
- **YAMAMOTO, T. ; TAYA, S. ; KAIBUCHI, K.** *Journal of Biochemistry,* 1999, vol. 126 (5), 799-803
- **BRODT, P ; SAMANI, A. ; NAVAB, R.** *Biochemical Pharmacology,* 2000, vol. 60, 1101-1107
- **MASSAGUE, J. ; WEIS-GARCIA, F.** *Cancer Surveys,* 1996, vol. 27, 41-64
- **PHILIP, P.A. ; HARRIS, A.L.** *Cancer Treatment and Research.,* 1995, vol. 78, 3-27
- **LACKEY, K. et al.** *Bioorganic and Medicinal Chemistry Letters,* 2000, (10), 223-226
- **MARTINEZ-IACACI, L. et al.** *Int. J. Cancer,* 2000, vol. 88 (1), 44-52
- **ABRAHAM, R.T.** *Current Opinion in Immunology,* 1996, vol. 8 (3), 412-8
- **CANMAN, C.E. ; LIM, D.S.** *Oncogene,* 1998, vol. 17 (25), 3301-3308
- **JACKSON, S.P.** *International Journal of Biochemistry and Cell Biology.,* 1997, vol. 29 (7), 935-8
- **ZHONG, H. et al.** *Cancer res,* 2000, vol. 60 (6), 1541-1545
- **POWIS, G. ; KOZIKOWSKI A.** 1994 New Molecular Targets for Cancer Chemotherapy. CRC press, 1994
- **SCHAROVSKY, O.G. ; ROZADOS, V.R. ; GERVASONI, S.I. ; MATAR, P.** *Journal of Biomedical Science,* 2000, vol. 7 (4), 292-8
- **ASHBY, M.N.** *Current Opinion in Lipidology,* 1998, vol. 9 (2), 99-102
- **BENNETT, C.F. ; COWSERT, L.M.** *BioChim. Biophys. Acta,* 1999, vol. 1489 (1), 19-30
- **GREEN, M.C. et al.** Monoclonal Antibody Therapy for Solid Tumors. *Cancer Treat. Rev.,* 2000, vol. 26 (4), 269-286
- Tyrosine Kinase Signalling in Breast cancer:erbB Family Receptor Tyrosine Kniases. *Breast cancer Res.,* 2000, vol. 2 (3), 176-183
- **BREKKEN, R.A. et al.** Selective Inhibition of VEGFR2 Activity by a monoclonal Anti-VEGF antibody blocks tumor growth in mice. *Cancer Res.,* 2000, vol. 60, 5117-5124
- **BRUNS CJ et al.** *Cancer Res.,* 2000, vol. 60, 2926-2935
- **SCHREIBER AB ; WINKLER ME ; DERYNCK R.** *Science,* 1986, vol. 232, 1250-1253
- **YEN L et al.** *Oncogene,* 2000, vol. 19, 3460-3469
- **REILLY RT et al.** *Cancer Res.,* 2000, vol. 60, 3569-3576
- **CHEN Y ; HU D ; ELING DJ ; ROBBINS J ; KIPPS TJ.** *Cancer Res.,* 1998, vol. 58, 1965-1971
- **WATER JS et al.** *J. Clin. Oncol.,* 2000, vol. 18, 1812-1823
- **KITADA S et al.** *Antisense Res. Dev.,* 1994, vol. 4, 71-79
- **ROSANIA et al.** *Exp. Opin. Ther. Patents,* 2000, vol. 10 (2), 215-230
- **KRIPPL et al.** *Int J Cancer,* 2003, vol. 106, 468
- **KATAOKA et al.** *Cancer Epidemiol Biomarkers Prev,* 2006, vol. 15, 1148
- **SPRAGGS et al.** *J Clin Oncol,* 2011, vol. 29, 667
- **FORMENTO et al.** *Pharmacogenomics,* 2009, vol. 10, 1277
- **KATAOKA et al.** *Cancer Epidemiol Biomarker Prev,* 2006, vol. 15, 1148